# EUROPEAN PATENT APPLICATION

(11) **EP 4 342 368 A1**
(43) Date of publication of application: **27.03.2024**
(21) Application number: 23199825.3
(22) Date of filing: 26.09.2023
(51) Int. Cl.: A61B 5/0205, A61B 5/33, A61B 5/339, A61B 5/352, A61B 5/00, A61B 5/332

(54) **SELECTIVELY DISPLAYING FILTERED PHYSIOLOGICAL PARAMETERS**

(30) Priority: 26.09.2022 US 202263410112 P
(71) Applicant: Stryker Corporation, Kalamazoo, MI 49002 (US)
(72) Inventor: DINH, Doan Huu, Kalamazoo, 49002 (US); TAYLOR, Tyson G., Kalamazoo, 49002 (US)
(74) Representative: V.O.

(57) **Abstract**

An example method is performed by a medical device and includes detecting an analog signal indicating a physiological parameter of a subject; converting the analog signal to first data; and determining a treatment parameter characterizing a treatment administered to the subject by a second medical device. The method further includes generating a filter characterized by the treatment parameter; generating second data by applying the filter to the first data; and displaying, on a user interface, the first data and the second data, wherein the second data is emphasized on the user interface.

## Description

### BACKGROUND

Medical devices can be used to facilitate patient monitoring, facilitate patient treatments, or a combination of both. In some environments, multiple medical devices are located in close proximity to one another. Some may be monitoring and/or treating the same patient, and some may be monitoring and/or treating different patients. In some cases, one medical device is administering a treatment that causes an artifact in a physiological parameter detected by another medical device.

### SUMMARY

Various implementations described herein relate to generating filtered data by removing an artifact in unfiltered data indicative of a physiological parameter detected by a monitoring device, wherein the artifact is caused by a treatment administered by a treating device. In particular cases, the filtered data is selectively displayed with the unfiltered data. For example, a waveform representing the filtered data is displayed at a greater emphasis than a waveform representing the unfiltered data.

Various medical devices are configured to perform artifact removal and/or filtering of data indicative of physiological parameters. In particular examples, a monitor-defibrillator removes a chest compression artifact from an electrocardiogram (ECG) of a patient. In many cases, the filtered data enables the medical device and/or a user (e.g., a rescuer or clinician) to more accurately assess the patient's condition. However, in some cases, the filtered data actually obscures the patient's condition. For instance, the filtered data can cause the medical device and/or the user to determine that the patient has a pathologic condition when no pathologic condition is present, or vice versa. Thus, in some cases, the filtered data can prevent the medical device, or the user, from accurately assessing the condition of the patient.

According to implementations of the present disclosure, the medical device outputs both filtered and unfiltered data for review by the user. In some cases, the filtered data is output at a greater emphasis than the unfiltered data, such as in conditions where the filtered data is more likely to accurately reflect the condition of the patient. In some instances, the user can control what data is emphasized. In various implementations, the medical device further de-emphasizes and/or removes the filtered data from the consideration of the user when the filtered data does not accurately reflect the condition of the patient.

According to an aspect is provided a method performed by a first medical device, the method including: detecting an analog signal indicating a physiological parameter of a subject; converting the analog signal to first data; determining a treatment parameter characterizing a treatment administered, e.g. to the subject, e.g. by a second medical device; generating a filter characterized by the treatment parameter; generating second data by applying the filter to the first data; and displaying, on a user interface, the first data and the second data, wherein the second data is emphasized on the user interface. Hence, monitoring the physiological parameter is rendered easier and less error-prone to a human observer. By providing the emphasized second data it is easier to concentrate on the second date, whereas the unfiltered first data can still available for monitoring so as to provide a possibility for human verification.

Optionally, the physiological parameter includes an electrocardiogram (ECG), a capnograph, a transthoracic impedance, a force administered to a user, a blood pressure, an airway parameter, a partial pressure of oxygen, an electroencephalogram (EEG), a temperature, a blood flow, or a pulse rate.

Optionally, determining the treatment parameter includes determining by the first medical device, by analyzing the first data, a frequency of the treatment. Alternatively, or additionally, determining the treatment parameter includes receiving by the first medical device, from the second medical device, a signal indicating the treatment parameter.

Optionally, the method includes, determining by the first medical device that the second data is more reliable than the first data.

Optionally, displaying the first data and the second data includes overlaying and time-aligning the first data and the second data on the user interface.

Optionally, displaying the first data and the second data includes: displaying a first waveform representing the first data; and displaying a second waveform representing the second data. The first waveform can include a different color than the second waveform, the first waveform can include a different contrast than the second waveform, the first waveform can include a different line size than the second waveform, the first waveform can include a different transparency than the second waveform, and/or the first waveform can include a different line style than the second waveform.

Optionally, the physiological parameter includes a first physiological parameter. The method can further include detecting a second physiological parameter of the subject; generating third data indicative of the second physiological parameter; and displaying, on the user interface, a waveform representing the third data.

Optionally, the method further includes detecting, by analyzing the first data or the second data, an event indicative of the treatment; and displaying, on the user interface, a symbol indicating the event.

Optionally, the first medical device is an external defibrillator. Optionally the first medical device is a monitor defibrillator.

According to an aspect is provided an external defibrillator including: a discharge circuit configured to administer a first treatment to a subject; a sensor configured to detect a physiological parameter of the subject; a display; and a processor configured to: generate first data indicating the physiological parameter of the subject; determine a treatment parameter characterizing a second treatment administered to the subject; generate a filter characterized by the treatment parameter; generate second data by applying the filter to the first data; and cause the display to visually present the first data at a first emphasis and the second data at a second emphasis.

Optionally the processor is configured for causing the display to visually present the first data and the second data by: causing the display to visually present a first waveform representing the first data; and causing the display to visually present a second waveform representing the second data.

Optionally, the second emphasis is greater than the first emphasis. Optionally, the first waveform includes a different color than the second waveform, the first waveform includes a smaller contrast than the second waveform, the first waveform includes a smaller line size than the second waveform, the first waveform includes a greater transparency than the second waveform, and/or the first waveform includes a different line style than the second waveform.

Optionally, the treatment parameter includes a frequency of the treatment, a time at which the treatment begins being administered to the subject, or a time at which the treatment stops being administered to the subject.

Optionally, the processor is further configured to: detect, by analyzing the first data or the second data, an event including a chest compression, a ventilation, or a QRS sense event; and display, on the user interface, a symbol indicating the event.

Optionally, the external defibrillator further includes a transceiver configured to receive, from a medical device administering the second treatment, a signal indicating the treatment parameter.

Optionally, the processor is configured to determine the treatment parameter by analyzing the first data or the second data.

According to an aspect is provided a monitor-defibrillator, including: a detection circuit configured to detect an electrocardiogram (ECG) signal of a subject; an analog-to-digital converter (ADC) configured to convert the ECG signal into unfiltered ECG data; a transceiver configured to receive a signal from a mechanical chest compression device, the signal indicating a frequency at which the mechanical chest compression device is administering chest compressions to the subject; a display; and a processor configured to: generate a comb filter that rejects the frequency and harmonics of the frequency; generate filtered ECG data by applying the comb filter to the unfiltered ECG data; cause the display to visually present the unfiltered ECG data at a first emphasis; and cause the display to visually present the filtered ECG data at a second emphasis, the second emphasis being greater than the first emphasis.

Optioanlly, the filtered ECG data is first filtered ECG data. Optionally, the processor is further configured to: determine, by analyzing the unfiltered ECG data or the filtered ECG data, a transition between chest compressions administered by the mechanical chest compression device to chest compressions administered by a rescuer; generate second filtered ECG data by applying an alternate filter to the unfiltered ECG data detected during the chest compressions administered by the rescuer; and cause the display to visually present the second filtered ECG data at a third emphasis, the third emphasis being different than the second emphasis.

Optionally, the processor is further configured to: detect, by analyzing the unfiltered ECG data or the filtered ECG data, a chest compression artifact in the unfiltered ECG data; and cause the display to visually present a symbol indicating a time at which the chest compression artifact is in the unfiltered ECG data.

According to an aspect is provided a monitor-defibrillator, including: a detection circuit configured to detect an electrocardiogram (ECG) signal of a subject; an analog-to-digital converter (ADC) configured to convert the ECG signal into unfiltered ECG data; a transceiver configured to receive a signal from a mechanical chest compression device, the signal indicating a frequency at which the mechanical chest compression device is administering chest compressions to the subject; a display configured to visually present a user interface element corresponding to an ECG lead; an input device configured to receive an input signal from a user; and a processor configured to: generate a comb filter that rejects the frequency and harmonics of the frequency; generate filtered ECG data by applying the comb filter to the unfiltered ECG data; cause the display to visually present the unfiltered ECG data in the user interface element at a first emphasis; cause the display to visually present the filtered ECG data in the user interface element at a second emphasis, the second emphasis being greater than the first emphasis; and in response to the input device receiving the input signal from the user: cause the display to visually present the unfiltered ECG data in the user interface element at a third emphasis; and cause the display to visually present the filtered ECG data in the user interface element at a fourth emphasis, the third emphasis being greater than the fourth emphasis.

Optionally, the display is configured to overlay and time-align the filtered ECG data and the unfiltered ECG data in the user interface element.

Optionally, the processor is further configured to: detect, based on the unfiltered ECG data or the filtered ECG data, an event including a chest compression, a ventilation, or a QRS sense event; and cause the display to visually present a symbol indicating the event.

Optionally, the processor is further configured to: cause the display to visually present a selectable element; and in response to the input device receiving a second input signal associated with the selectable element, cause the display to: visually present the filtered ECG data without the unfiltered ECG data; or visually present the unfiltered ECG data without the filtered ECG data.

Optionally, the processor is further configured to: detect a filter artifact in the filtered ECG data; and cause the display to visually present: the filter artifact at a fifth emphasis; or an indication of the filter artifact.

Optionally, causing the display to visually present the unfiltered ECG data at the third emphasis includes causing the display to visually present a first waveform representing the unfiltered ECG, and wherein causing the display to visually present the filtered ECG data at the fourth emphasis includes causing the display to visually present a second waveform representing the filtered ECG, the second waveform having a smaller line size than the first waveform.

According to an aspect is provided a method, performed by a first medical device, including: detecting an analog signal indicating a physiological parameter of a subject; converting the analog signal to first data; determining a treatment parameter characterizing a treatment administered to the subject; generating a filter characterized by the treatment parameter; generating second data by applying the filter to the first data; displaying the first data at a first emphasis; displaying the second data at a second emphasis, the second emphasis being greater than the first emphasis; determining that the first data is more reliable than the second data; and in response determining that the first data is more reliable than the second data: displaying the first data at the second emphasis; and displaying the second data at the first emphasis.

Optionally, the physiological parameter includes an electrocardiogram (ECG) of the subject, and the treatment includes chest compressions administered to the subject.

Optionally, determining that the first data is more reliable than the second data includes determining that an artifact associated with the treatment is absent from the first data.

Optionally, displaying the first data at the first emphasis includes displaying a first waveform representing the first data; and displaying the second data at the second emphasis includes displaying a second waveform representing the second data.

Optionally, the first waveform includes a different color than the second waveform, the first waveform includes a different contrast than the second waveform, the first waveform includes a different line size than the second waveform, the first waveform includes a different transparency than the second waveform, and/or the first waveform includes a different line style than the second waveform.

Optionally, the treatment parameter includes a frequency of the treatment, a time at which the treatment began being administered to the subject, or a time at which the treatment stops being administered to the subject.

Optionally, the further includes detecting by the first medical device, by analyzing the first data or the second data, an event including a chest compression, a ventilation, or a QRS sense event; and displaying, on the user interface, a symbol or a letter indicating the event.

According to an aspect is provided a medical device, including: a sensor configured to detect an analog signal indicating a physiological parameter of a subject; an analog-to-digital converter (ADC) configured to convert the analog signal to first data; and a processor configured to: generate a filter configured to remove an artifact associated with a treatment administered to the subject; generate second data by applying the filter to the first data; determine that the first data is more reliable than the second data; and in response to determining that the first data is more reliable than the second data: cause a user interface, such as a display, to visually present a first waveform representing the first data at a first emphasis; and cause the user interface to visually present a second waveform representing the second data at a second emphasis, the first emphasis being greater than the second emphasis.

Optionally, the physiological parameter includes an electrocardiogram (ECG), a capnograph, a force administered to the subject, a blood pressure, an airway parameter, a partial pressure of oxygen, an electroencephalogram (EEG), a temperature, a blood flow, a pulse rate, or a transthoracic impedance.

Optionally, the processor is further configure for detecting, by analyzing the first data or the second data, an event including a chest compression, a ventilation, or a QRS sense event; and causing the user interface to display a symbol indicating the event.

Optionally, the treatment administered to the subject is administered by another medical device.

Optionally, the sensor being a first sensor, the physiological parameter being a first physiological parameter, the medical device further includes: a second sensor configured to detect a second physiological parameter, wherein the processor is further configured to cause the user interface to visually present third data representing the second physiological parameter.

Optionally, the processor is configured to cause the user interface to visually present the third data by visually presenting a third waveform at a third emphasis.

Optionally, the first waveform includes a different color than the second waveform and the third waveform, the first waveform includes a different contrast than the second waveform and the third waveform, the first waveform includes a different line size than the second waveform and the third waveform, the first waveform includes a different transparency than the second waveform and the third waveform, and/or the first waveform includes a different line style than the second waveform and the third waveform.

According to an aspect is provided a method, including: generating filtered data by removing a treatment artifact from unfiltered data, the unfiltered data representing a physiological parameter of a subject; displaying a segment of the filtered data corresponding to a treatment time interval, a treatment being administered to the subject during the treatment time interval; and displaying a segment of the unfiltered data corresponding to a non-treatment time interval, the treatment being paused during the non-treatment time interval.

Optionally, the method further includes: identifying a filter artifact in the segment of the filtered data corresponding to the treatment time interval, wherein displaying the segment of the filtered data corresponding to the treatment time interval includes indicating the filter artifact.

It will be appreciated that any of the aspects, features and options described herein can be combined. It will particularly be appreciated that any of the aspects, features and options described in view of the medical devices apply equally to the methods, and vice versa.

### BRIEF DESCRIPTION OF THE DRAWINGS

The detailed description is described with reference to the accompanying figures. In the figures, the left-most digit(s) of a reference number identifies the figure in which the reference number first appears. The use of the same reference numbers in different figures indicates similar or identical components or features. This application contains at least one drawing that is represented in color.
FIG. 1 illustrates an example environment for selectively outputting processed data to a rescuer.
FIGS. 2A to 2C illustrate different examples of emphasized lines. In various implementations, a medical device displays multiple waveforms simultaneously, such as waveforms representing filtered and unfiltered data.
FIG. 3 illustrates an example display of a medical device that visually presents various physiological event symbols in addition to parameter waveforms.
FIG. 4 illustrates an example display of a medical device that visually presents various physiological event symbols in addition to parameter waveforms.
FIG. 5 illustrates an example of unprocessed ECG detected by a medical device and processed ECG displayed by the medical device.
FIGS. 6A to 6C illustrate various examples of processed ECG displayed by a medical device.
FIG. 7 illustrates an example process for simultaneously displaying filtered and unfiltered data.
FIG. 8 illustrates an example process for emphasizing one type of displayed data over another type of displayed data.
FIG. 9 illustrates an example of an external defibrillator configured to perform various functions described herein.
FIG. 10 illustrates a chest compression device configured to perform various functions described herein.

### DETAILED DESCRIPTION

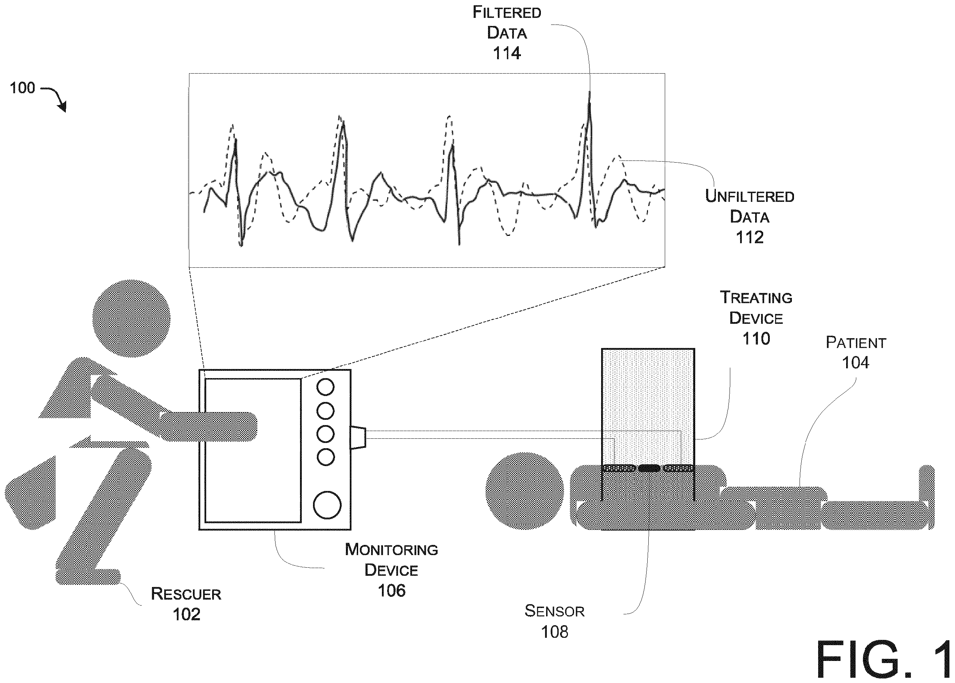

FIG. 1 illustrates an example environment 100 for selectively outputting processed data to a rescuer 102. In various implementations, the environment 100 includes a rescue scene. The rescue scene, for instance, is in a clinical environment (e.g., a hospital) or a non-clinical environment (e.g., the scene of an accident).

In various cases, the rescuer 102 is treating a patient 104. For instance, the rescuer 102 is an EMT professional monitoring and/or treating a medical condition of the patient 104. In some cases, the patient 104 is experiencing cardiac arrest, respiratory arrest, or some other dangerous medical condition.

The rescuer 102 monitors the condition of the patient 104 using a monitoring device 106. For instance, the monitoring device 106 may be a monitor-defibrillator, a medical imaging device, an ultrasound monitor, a standalone ECG monitor, or another type of patient monitor. The monitoring device 106 includes and/or is communicatively coupled to a sensor 108. The sensor 108 is configured to detect at least one physiological parameter of the patient 104. According to various implementations, the signal detected by the sensor 108 is referred to as a "parameter signal." Examples of physiological parameters include, for instance, an ECG, an impedance, a force administered to the patient 104, a blood pressure, an airway parameter (e.g., a partial pressure of carbon dioxide, a partial pressure of oxygen, a capnograph, an end tidal gas parameter, a flow rate, etc.), a blood oxygenation (e.g., a pulse oximetry value, a regional oximetry value, etc.), an electroencephalogram (EEG), a temperature, a heart sound, a blood flow rate, a physiological geometry (e.g., a shape of a blood vessel, an inner ear shape, etc.), a heart rate, a pulse rate, or the like. For example, the sensor 108 includes at least one of electrodes, a detection circuit, defibrillator pads, a force sensor, a blood pressure cuff, an ultrasound-based blood pressure sensor, an invasive (e.g., intra-arterial) blood pressure sensor (e.g., including a cannula inserted into the patient 104), a gas sensor (e.g., a carbon dioxide and/or oxygen sensor), a flowmeter, a pulse oximetry sensor a regional oximetry sensor, a thermometer, a microphone, an ultrasound transducer, a medical imaging device (e.g., an ultrasound imaging device), or the like. In various cases, the monitoring device 106 outputs the physiological parameter(s) to the rescuer 102. For instance, the monitoring device 106 includes a display, a speaker, or haptic feedback device that conveys the physiological parameter(s) to the rescuer 102.

A treating device 110 (also referred to as a "treatment device") administers a treatment to the patient 104. For example, the treating device 110 is a monitor-defibrillator, an automated external defibrillator (AED), mechanical chest compression device, a smart bag-valve mask, a ventilator, a heart-lung machine, an intravenous fluid (IV) pump, or the like. Examples of treatments include defibrillation, pacing, cardioversion, administration of chest compressions, administration of oxygen to the airway of the patient 104, movement of air in the airway of the patient, administration of fluids to the patient 104, extracorporeal membrane oxygenation (ECMO), administration of a medication to the patient 104, or the like. In some implementations, the monitoring device 106 is also configured to administer a treatment to the patient 104. Further, in some cases, the treating device 110 is configured to detect one or more physiological parameters of the patient 104.

The monitoring device 106, in some cases, detects the treatment administered by the treating device 110 by analyzing the parameter signal. For instance, the monitoring device 106 identifies the treatment based on a time, frequency, or shape of an artifact in the parameter signal that corresponds to the treatment. For instance, a chest compression artifact in an ECG can be recognized as a periodic spike in the ECG at a frequency consistent with chest compression administration. In various implementations, the monitoring device 106 identifies the type of device administering the treatment based on the artifact. For example, a chest compression artifact produced one type of mechanical chest compression device is shaped differently than a chest compression artifact produced by another type of mechanical chest compression device. The monitoring device 106, in some cases, determines a parameter of the treatment by analyzing the signal detected by the sensor 108. This type of parameter, as described herein, can be referred to as a "treatment parameter." Examples of treatment parameters include timing, frequency, shape, or magnitude of the treatment.

In various implementations, the monitoring device 106 and the treating device 110 are communicatively coupled to one another. In particular examples, the monitoring device 106 or the treating device 110 reports a detected physiological parameter to the other device. For instance, the first treating device 110 may include a blood pressure sensor and may report a blood pressure of the patient 104 to the monitoring device 106. In some cases, the monitoring device 106 or the treating device 110 reports a treatment parameter to the other device. In some examples, the treating device 110 reports a frequency of chest compressions administered by the treating device 110 to the monitoring device 106. The receiving device may perform one or more actions based on the physiological parameter and/or the treatment parameter. Actions performed by the monitoring device 106 or the treating device 110 include initiating a measurement of a physiological parameter at a particular time or frequency, outputting a signal to the rescuer 102, outputting a signal to the patient 104, performing a treatment at a particular time or frequency, adjusting a treatment parameter of an ongoing treatment, or any combination thereof. According to some implementations, the monitoring device 106 or the treating device 110 instructs the other device to perform one or more actions. The receiving device, in turn, performs the action(s) based on the instruction from the monitoring device 106 or the first treating device 110. For instance, the monitoring device 106 instructs the treating device 110 to cease administering chest compressions to the patient 104 at a predetermined time, and the monitoring device 106 administers a defibrillation shock to the patient 104 at the particular time. By exchanging reports, instructions, or other data, the monitoring device 106 and the treating device 110 can coordinate monitoring and treatment of the patient 104.

To exchange data, the monitoring device 106 and/or the treating device 110 are configured to establish and/or communicate via a communication channel. As used herein, the term "communication channel," and its equivalents, may refer to a medium over which a first endpoint (e.g., a sender) transmits information to one or more second endpoints (e.g., receivers). Examples of communication channels include wired connections, such as Ethernet or fiber optic paths, as well as wireless connections, such as Institute of Electronics and Electrical Engineers (IEEE) (e.g., WI-FI, BLUETOOTH, etc.) or 3^{rd} Generation Partnership Program (3GPP) (e.g., Long Term Evolution (LTE), New Radio (NR), etc.) connections. As used herein, the term "endpoint," and its equivalents, may refer to an entity that is configured to transmit and/or receive data. Examples of endpoints include user equipment (UE) (e.g., mobile phones, tablet computers, etc.), computers, base stations, access points (APs), servers, compute nodes, medical devices, Internet of Things (IoT) devices, and the like.

In some implementations, the communication channel between the monitoring device 106 and the treating device 110 is established when the monitoring device 106 and the treating device 110 are paired. In particular cases, the monitoring device 106 and treating device 110 refrain from sharing substantive data (e.g., physiological metrics, reports about the patient 104, instructions for treating the patient 104, etc.) until the monitoring device 106 and the treating device 110 are paired. As used herein, the term "paired," and its equivalents, may refer to a state of multiple devices that have a shared link key that enables each device to cryptographically authenticate data it receives from any other device among the multiple devices.

In particular cases, a first paired device encrypts data prior to transmitting the data to a second paired device, and the second paired device restores the original data by decrypting the encrypted data. As used herein, the term "encrypt," and its equivalents, refers to a process of translating data from one format (e.g., an unencoded format) into an encoded format. In various cases, the encoded format is referred to as "ciphertext." Unencoded data, which has not been encrypted, may be referred to as being in "plaintext." In various examples, an entity encrypts data using at least one encryption key. An encryption key is a parameter that defines the translation of data from the one format into the encoded format. As used herein, the term "decrypt," and its equivalents, refers to a process of translating data from an encoded format into another format (e.g., an unencoded format), such as a plaintext format. In various examples, an entity encrypts data using at least one decryption key. A decryption key is a parameter that defines the translation of data from the encoded format into the other format. A link key, for example, is an encryption and/or decryption key.

Various cryptographic techniques can be utilized in accordance with the features described in this disclosure. For example, data can be encrypted and decrypted via a symmetric key, wherein the encryption key and the decryption key are equivalent. In some cases, data can be encrypted and decrypted via asymmetric keys, wherein the encryption key and the decryption key are different. Cryptographic hash functions (CHFs) are examples of cryptographic techniques. Examples of cryptographic techniques include the Data Encryption Standard (DES), Advanced Encryption Standard (AES), Elliptic Curve Cryptography (ECC), Rivest-Shamir-Adleman (RSA), Secure Hash Algorithm (SHA)-1, SHA-2, SHA-3, BLAKE, BLAKE2, BLAKE3, WHIRLPOOL, MD2, MD4, MD5, MD6, Temporal Key Integrity Protocol (TKIP), Rivest cipher 4 (RC4), variably modified permutation composition (VMPC), blowfish, Twofish, Threefish, Tiny Encryption Algorithm (TEA), Extended TEA (XTEA), Corrected Block TEA (XXTEA), Diffie-Hellman exchange (DHE), elliptic curve DHE, supersingular isogeny Diffie-Hellman (SIDH) key exchange, and so on. Any suitable encryption or decryption technique can be used in accordance with implementations of this disclosure.

In various implementations of the present disclosure, the monitoring device 106 generates unfiltered data 112 based on the physiological parameter detected by the sensor 108. For example, the monitoring device 106 includes an analog-to-digital converter (ADC) that converts a signal representing the physiological parameter into digital data. However, the patient 104 is simultaneously receiving a treatment from the treating device 110. In various cases, the treatment generates an artifact in the unfiltered data 112. For instance, the unfiltered data 112 represents an ECG of the patient 104 while the patient 104 is receiving chest compressions from the treating device 110, such that the unfiltered data 112 includes a chest compression artifact.

The artifact in the unfiltered data 112 may impede review of the physiological parameter by the rescuer 102 and/or the monitoring device 106. In some cases, the rescuer 102 is unable to identify whether the patient 104 is experiencing a dangerous medical condition due to the presence of the artifact. For example, the rescuer 102 may be unable to determine whether the patient 104 is experiencing an arrhythmia treatable by defibrillation (also referred to as a "shockable arrhythmia"), such as ventricular fibrillation (VF) or pulseless ventricular tachycardia (VT), due to the presence of the chest compression artifact in the unfiltered data 112 representing the ECG of the patient 104. While the rescuer 102 could temporarily pause the chest compressions by operating the treating device 110, and view the unfiltered data 112 without the chest compression artifact, such a pause in chest compressions could harm the patient 104. Thus, it is preferred to avoid pauses in chest compressions and other treatments during rescue events.

In various implementations of the present disclosure, the monitoring device 106 is configured to generate filtered data 114 by at least partially removing the artifact from the unfiltered data 112. In some examples, the monitoring device 106 applies a filter to the unfiltered data 112, such as an adaptive filter (e.g., a Wiener filter, a Kalman filter, or the like), an nth order filter (e.g., a zero-th order filter) a comb filter, an inverse comb filter, a high-pass filter, a band reject filter, a finite impulse response (FIR) filter, an infinite impulse response (IIR) filter, or a combination thereof. In some cases, the monitoring device 106 converts the unfiltered data 112 from the time domain into the frequency (e.g., a Fourier) domain, a Laplace domain, a Z-transform domain, or a wavelet (e.g., a continuous wavelet transform, a discrete wavelet transform, etc.) domain, and removes at least a portion of the artifact by processing the converted data. According to some examples, the monitoring device 106 identifies and subtracts the artifact from the unfiltered data 112. In some instances, the monitoring device 106 identifies and subtracts the artifact based on the detected treatment. For example, the monitoring device 106 detects another signal indicative of the treatment, such as compression data from a chest compression sensor, cross-correlates the unfiltered data 112 with the data corresponding to the treatment (e.g., the impedance, the acceleration of a separate compression detector, the velocity of the compression detector, etc.), identifies the artifact based on the cross-correlation, and subtracts the artifact from the unfiltered data 112. In some instances, the monitoring device 106 denoises the unfiltered data 112. For example, the monitoring device 106 removes at least a portion of the artifact by performing spectral subtraction on the unfiltered data 112.

In some implementations, the artifact removal and/or filtering technique applied by the monitoring device 106 is associated with a latency period. As used herein, the term "latency period" refers to an interval between a time at which a filter is initially applied to unfiltered data and a time at which an artifact is substantially removed from the unfiltered data by the filter. Several factors contribute to the latency period of a filter. Adaptive filters, for instance, are associated with a greater latency period than non-adaptive filters, because they may be configured to learn the artifact during the latency period. Artifact removal and/or filtering techniques that require conversion to a separate domain, such as the Fourier domain, may also be associated with a longer latency period than alternative techniques. Various types of digital filters are associated with different types of non-zero latency periods.

Optionally, the monitoring device 106 applies additional filtering techniques to reduce the harmonics of the artifact in the selected segment of the unfiltered data 112. In particular cases, the monitoring device 106 generates the filtered data 114 by applying a comb filter or multiple notch filters to the unfiltered data 112. In various instances, comb filters are particularly suited to remove chest compression artifacts administered by a mechanical chest compression device. In some cases, an example comb filter rejects a band including the frequency of the chest compressions as well as one or more harmonics of the frequency. For example, the monitoring device 106 applies a comb filter with multiple stopbands that correspond to the fundamental frequency of the treatment administered to the patient 104 and one or more harmonics of the fundamental frequency. In some implementations, the treating device 110 reports the frequency of the chest compressions to the monitoring device 106 over the communication channel, and the monitoring device 106 generates and/or adjusts the filter applied to the unfiltered data 112 accordingly. In some implementations, the monitoring device 106 identifies the frequency of the chest compressions by analyzing the unfiltered data 112 itself.

In various examples, the monitoring device 106 displays the filtered data 114 to the rescuer 102. However, in some cases, the filtered data 114 can mislead and/or confuse the rescuer 102. For instance, a rescuer 102 may be used to seeing the artifact when the treatment is being administered to the patient 104, and may be concerned that the monitoring device 106 is malfunctioning if the artifact is not visible to the rescuer 102. In some cases, the filter used to generate the filtered data 114 is associated with a latency period, wherein the artifact substantially remains in the filtered data 114 (or the filter temporarily introduces additional artifact) until the latency period has elapsed after the filter is applied to the unfiltered data 112. In some examples, the filtered data 114 is less reliable than the unfiltered data 112. As used herein, the term "reliable," and its equivalents, can refer to data that accurately represents a condition of a patient. For instance, if the treating device 110 temporarily pauses administering the treatment to the patient 104, but the monitoring device 106 continues to apply the filter to the unfiltered data 112, then the filtered data 114 will be noisier and/or less representative of the condition of the patient 104 than the unfiltered data 112. In some examples in which the treating device 110 alters the treatment administered to the patient 104 (e.g., the treating device 110 changes a frequency of ventilations administered to the patient 104), but the monitoring device 106 continues to apply the filter optimized to the original treatment parameter, then the filtered data 114 will be noisier and/or less representative of the condition of the patient 104 than the unfiltered data 112.

According to various implementations of the present disclosure, the monitoring device 106 simultaneously displays the unfiltered data 112 and the filtered data 114 to the rescuer 102. For example, a display of the monitoring device 106 visually presents a first waveform representing the unfiltered data 112 and a second waveform representing the filtered data 114. In some examples, the first waveform overlaps and/or is time-aligned with the second waveform. Accordingly, the rescuer 102 can manually observe the unfiltered data 112 and the filtered data 114 at the same time. In cases where the unfiltered data 112 or the filtered data 114 is less reliable than the other type of data, the rescuer 102 may manually review the more reliable type of data in order to accurately assess the condition of the patient 104.

In some cases, the monitoring device 106 emphasizes one type of data over another type of data visually presented on the display. For instance, the monitoring device 106 emphasizes a first waveform representing the unfiltered data 112 differently than a second waveform representing the filtered data 114. The waveforms, for instance, have different colors, transparencies, line styles (e.g., dashed, dotted, or solid line styles), boldness, contrast, widths, blinking patterns, or any combination thereof. In some cases, one waveform has a greater emphasis than the other waveform when it is displayed with a predetermined color, a lower transparency, a predetermined line style (e.g., a solid line style), a greater boldness, a greater contrast, a larger width, without blinking, or any combination thereof.

The monitoring device 106 displays the filtered data 114 at the greater emphasis when one or more conditions are satisfied. In some cases, the filtered data 114 is displayed at the greater emphasis when a latency of the filter has expired. According to some cases, the filtered data 114 is displayed at the greater emphasis in response to the monitoring device 106 determining that the treatment artifact is present in the unfiltered data 112. In some examples, the filtered data 114 is displayed at the greater emphasis when the treating device 110 communicates, to the monitoring device 106, that the treatment is being administered by the treating device 110 and/or that the treating device 110 has detected a physiological parameter from the patient 104 (e.g., indicating that the treating device 110 is physically coupled to the patient 104). In various cases, the monitoring device 106 displays the filtered data 114 at the greater emphasis when the filtered data 114 is more likely to represent the condition of the patient 104 than the unfiltered data 112.

However, under some conditions, the monitoring device 106 hides the filtered data 114 and/or displays the filtered data 114 at a lower emphasis than the unfiltered data 112. For example, the monitoring device 106 displays the unfiltered data 112 at the greater emphasis, or without the filtered data 114, during the latency period of the filter. In some examples, the monitoring device 106 displays the unfiltered data 112 at the greater emphasis, or without the filtered data, upon determining that the treating device 110 has discontinued and/or altered the treatment. The monitoring device 106 determines whether the treating device 110 has discontinued and/or altered the treatment based on analyzing the unfiltered data 112 and/or receiving a communication from the treating device 110.

In some cases, multiple treatments are administered to the patient 104 during a rescue event. According to various implementations, at least some of the treatments are administered at different times. In some examples, the treating device 110 administers a first treatment to the patient 104 during a first time interval and the rescuer 102 administers a second treatment to the patient 104 during a second time interval. For instance, the treating device 110 administers chest compressions or ventilations to the patient 104 during the first time interval and the rescuer 102 administers manual chest compressions or ventilations (e.g., using a bag-valve mask) during the second time interval. In some examples, the monitoring device 106 applies a first filter to the unfiltered data 112 to remove the artifact caused by the first treatment and applies a second filter to the unfiltered data 112 to remove the artifact caused by the second treatment. The first filter, for instance, includes a comb filter and the second filter includes a non-comb filter. In various implementations, the filtered data 114 includes a first segment corresponding to the application of the first filter and includes a second segment corresponding to the application of the second filter. The monitoring device 106, in some cases, displays the first segment at a different emphasis than the second segment.

In some implementations, the rescuer 102 changes the emphasis of the unfiltered data 112 and/or the filtered data 114 by interacting with the monitoring device 106. For example, the monitoring device 106 changes the relative emphases of the unfiltered data 112 and the filtered data 114 in response to receiving an input signal from the rescuer 102. In particular cases, the monitoring device 106 displays a user interface element that corresponds to a selectable option for changing the relative emphasis of the unfiltered data 112 and/or the filtered data 114. For instance, the monitoring device 106 includes a touchscreen displaying the user interface element, and when the monitoring device 106 detects the rescuer 102 touching the user interface element on the touchscreen, the monitoring device 106 changes the displayed emphasis of the unfiltered data 112 and/or the filtered data 114.

According to various implementations, the monitoring device 106 detects one or more events by analyzing the unfiltered data 112 and/or the filtered data 114. As used herein, the terms "events," "physiological events," and their equivalents may refer to a change in a condition of a patient, a discrete treatment administered to the patient, or a discrete function performed by the body of the patient. Examples of events include, for instance, a physiological parameter rising above a first threshold, the physiological parameter falling below a second threshold, a treatment administered to the patient, a non-continuous physiological parameter being detected from the patient, or any other thing that happens by or to the patient. In particular implementations of the present disclosure, the monitoring device 106 may detect the occurrence of individual chest compressions administered to the patient 104, the occurrence of individual ventilations administered to the patient 104, the occurrence of individual QRS complexes in the ECG of the patient 104, the occurrence of a return of spontaneous circulation (ROSC), the initiation of a particular type of arrhythmia (e.g., VF, VT, bradycardia, tachycardia, ventricular fibrillation, or asystole), the resolution of the particular type of arrhythmia, or the like. In some cases, the monitor 106 detects discrete physiological parameters, such as discrete blood pressure detected using a blood pressure cuff. QRS complex detection, for instance, is performed using an existing QRS sensing algorithm. QRS complex detection is performed on the unfiltered data 112, the filtered data 114, or both. For instance, a QRS sensing algorithm suitable for chest-compression artifacted ECG, or to artifacted ECG, may be implemented. Alternatively or in addition, a QRS sensing algorithm that is reliable for both unartifacted and artifacted ECG can be implemented. In some cases, a first QRS sensing algorithm is applied to the unfiltered data 112 when a predetermined artifact (e.g., a treatment artifact) is absent from the unfiltered data 112, and a second QRS sensing algorithm is applied to the filtered data 114 when the predetermined artifact is present in the unfiltered data 112.

In various implementations, the monitoring device 106 is configured to detect chest compressions, ventilations, or some other periodic treatment performed on the patient 104 by detecting a periodic artifact in the unfiltered data 112 and/or the filtered data 114. In some cases, the periodic artifact is represented by a predictable fundamental frequency and its harmonics. For instance, the periodic treatment is detected by applying a bandpass filter to the unfiltered data 112 that is centered around a therapeutically significant frequency at which the periodic treatment is applied. Chest compressions, for instance, may be detected in an ECG by applying a bandpass filter with cutoff frequencies of 1 to 3 Hz. Ventilations, in some examples, are detected in a transthoracic impedance by applying a bandpass filter with cutoff frequencies of 0.05 to 1 Hz. In some cases, the monitoring device 106 detects the treatment by determining that the periodic artifact conforms to a predetermined shape associated with the treatment.

In various implementations, ROSC is detected based on one or more physiological parameters. In some cases, ROSC is detected when a particular physiological parameter or combination of parameters exceeds a threshold. For instance, a physiological parameter indicative of blood flow can be a blood pressure, blood oxygenation, capnograph, EtCO2, or a score combining any combination thereof, that is above a predetermined threshold may be indicative of ROSC. In some cases, ROSC is detected by detecting blood flow directly. Blood flow, for instance, is detected during a treatment pause (e.g., a chest compression pause), by detecting a waveform of a physiological parameter indicative of blood flow, by detecting a sudden change in the waveform, or a combination thereof. For instance, a 0.5 to 10 fold increase in the physiological parameter over the course of 5 to 60 seconds is indicative of ROSC.

In some examples, the monitoring device 106 indicates one or more events. For example, the monitoring device 106 visually presents a symbol indicating the occurrence of an event using the display. In some cases, the symbol overlaps, or is displayed adjacent to, the unfiltered data 112 and/or the unfiltered data 114. For instance, the symbol is displayed at a position corresponding to the time at which the event occurred with respect to the time scale of the unfiltered data 112 and/or the unfiltered data 114.

In particular examples, the monitoring device 106 is a monitor-defibrillator and the treating device 110 is a mechanical chest compression device. The treating device 110 administers chest compressions to the patient 104. In these examples, the treating device 110 transmits at least one communication signal to the monitoring device 106 reporting when the chest compressions begin and/or end. Based on the communication signal(s), the monitoring device 106 outputs both the unfiltered data 112 and the filtered data 114 when the chest compressions are administered to the patient 104. Further, the monitoring device 106 refrains from outputting the filtered data 114 before the chest compressions begin and/or after the chest compressions end.

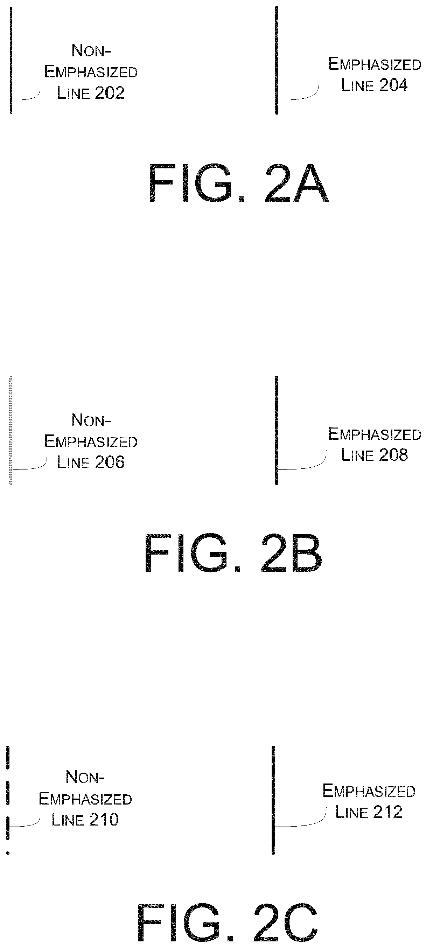

FIGS. 2A to 2C illustrate different examples of emphasized lines. In various implementations, a medical device (e.g., the monitoring device 106) displays multiple waveforms simultaneously, such as waveforms representing filtered and unfiltered data. To distinguish between the waveforms, the medical device displays the lines of the waveforms at different emphasis.

FIG. 2A illustrates a non-emphasized line 202 and an emphasized line 204 having different widths. In particular, the non-emphasized line 202 has a shorter width than the emphasized line 204.

FIG. 2B illustrates an non-emphasized line 206 and an emphasized line 208 having different colors, contrast, or transparencies. The non-emphasized line 206 has a different color than the emphasized line. For instance, the emphasized line 208 has a bolder, more pigmented color than the non-emphasized line 206. In some cases, the non-emphasized line 206 has a lower contrast with respect to a background than the emphasized line 208. In various examples, the non-emphasized line 206 has a greater transparency than the emphasized line 208.

FIG. 2C illustrates a non-emphasized line 210 and an emphasized line 212 having different line styles. In particular, the non-emphasized line 210 has a dashed line style, whereas the emphasized line has a solid line style.

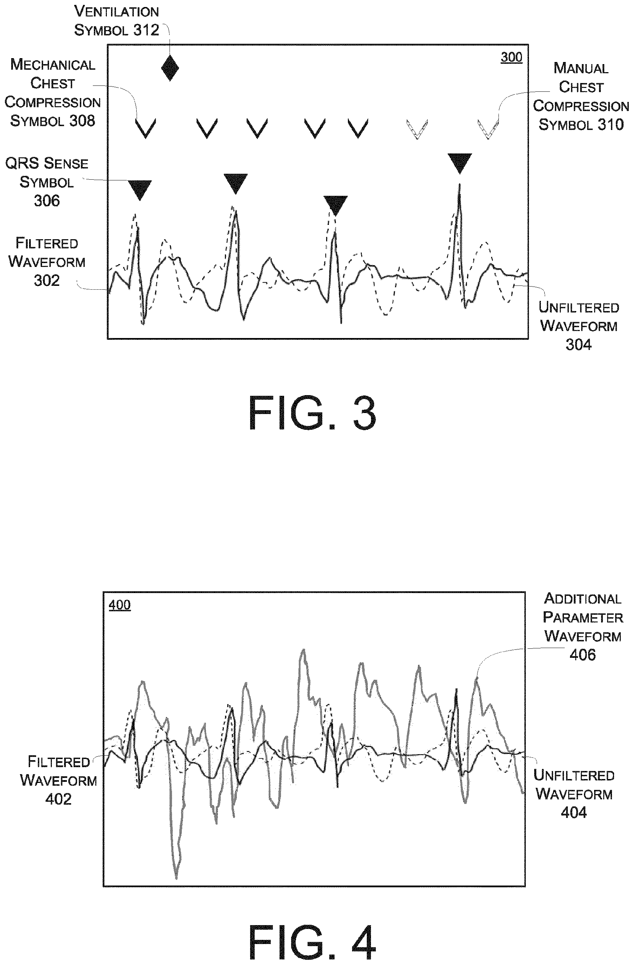

FIG. 3 illustrates an example display 300 of a medical device that visually presents various physiological event symbols in addition to parameter waveforms. In particular, the display 300 visually presents a filtered waveform 302 and an unfiltered waveform 304. The filtered waveform 302 and the unfiltered waveform 304 are overlapping and time-aligned on the display 300. For example, a magnitude of the filtered waveform 302 and the unfiltered waveform 304 may correspond to a vertical direction on the display 300, and a time dimension may correspond to a horizontal direction (e.g., left to right) on the display 300. The medical device may detect the unfiltered waveform 304 using one or more sensors. The medical device may generate the filtered waveform 302 by applying one or more filters to the unfiltered waveform 304. In the example of FIG. 3, the unfiltered waveform 304 represents an ECG of an individual receiving chest compressions, and the filtered waveform 302 represents the ECG with an artifact corresponding to the chest compressions at least partially removed. However, implementations of the present disclosure could utilize other types of physiological parameters. The filtered waveform 302 may be displayed at a greater emphasis than the unfiltered waveform 304.

Further, the medical device may detect physiological events and display symbols corresponding to the physiological events on the display 300. For example, the medical device detects QRS sense events in the filtered waveform 302 and displays QRS sense symbols 306 corresponding to the QRS sense events. In some cases, the medical device at least partially detects the QRS sense events by detecting local maxima within the filtered waveform 302 and/or in the (e.g., first) derivative of the filtered waveform 302. The QRS sense events are represented by triangles displayed above the R wave of individual QRS complexes within the filtered waveform 302.

In various implementations, the medical device detects chest compressions administered to the individual. In some cases, the chest compressions are detected by identifying peaks in the unfiltered waveform 304 and/or by detecting peaks in data representing a non-ECG physiological parameter, such as transthoracic impedance. Further, the medical device detects whether each chest compression is a mechanical chest compression (i.e., a chest compression administered by a mechanical chest compression device) or a manual chest compression (i.e., a chest compression administered by a person). In various cases, the medical device detects the type of an example chest compression by determining whether the shape of an artifact corresponding to the example chest compression (e.g., an artifact in the unfiltered waveform 304 or the non-ECG physiological parameter data) matches a predetermined shape corresponding to a mechanical chest compression and/or whether shape of the artifact matches a predetermined shape corresponding to a manual chest compression. The display 300 visually outputs a mechanical chest compression symbol 308 corresponding to the detected mechanical chest compressions and a manual chest compression symbol 310 corresponding to the detected manual chest compressions. In various cases, the mechanical chest compression symbol 308 and the manual chest compression symbol 310 are both v-shaped, but have different colors or fill patterns.

The medical device detects ventilations administered to the individual and visually presents a ventilation symbol 312 based on the detected ventilations. In some cases, the medical device detects the ventilations by analyzing the filtered waveform 302 and/or the unfiltered waveform 304. For instance, the medical device identifies artifacts in the unfiltered waveform 304 corresponding to the ventilations. In various examples, the medical device detects the ventilations based on non-ECG physiological parameter data, such as data representing a flow rate or a partial pressure of a gas (e.g., CO2, O2, etc.) in an airway of the individual. The ventilation symbol 312, in FIG. 3, is represented by a diamond shape.

In various implementations, the symbols representing the physiological events (i.e., the QRS sense symbol 306, the mechanical chest compression symbol 308, the manual chest compression symbol 310, and the ventilation symbol 312) are positioned on the display 300 to convey the time at which the physiological events are detected. For instance, the ventilation symbol 312 is placed at a horizontal position along the display 300 that corresponds to a portion of the unfiltered waveform 304 detected at the same time as the ventilation. Although the symbols illustrated in FIG. 3 have particular shapes, implementations of the present disclosure are not limited to the specific shapes illustrated.

FIG. 4 illustrates an example display 400 of a medical device that visually presents various physiological event symbols in addition to parameter waveforms. In particular, the display 400 visually presents a filtered waveform 402 and an unfiltered waveform 404. The filtered waveform 402 and the unfiltered waveform 404 are overlapping and time-aligned on the display 400. For example, a magnitude of the filtered waveform 402 and the unfiltered waveform 304 may correspond to a vertical direction on the display 400, and a time dimension may correspond to a horizontal direction (e.g., left to right) on the display 400. The medical device may detect the unfiltered waveform 404 using one or more sensors. The medical device may generate the filtered waveform 402 by applying one or more filters to the unfiltered waveform 404. In the example of FIG. 4, the unfiltered waveform 404 represents an ECG of an individual receiving chest compressions, and the filtered waveform 402 represents the ECG with an artifact corresponding to the chest compressions at least partially removed. The filtered waveform 402 may be displayed at a greater emphasis than the unfiltered waveform 404.

In addition, the display 400 outputs an additional parameter waveform 406. The additional parameter waveform 406 is a waveform representing a non-ECG physiological parameter. In the particular example provided by FIG. 4, the additional parameter waveform 406 represents a transthoracic impedance of the individual. In various cases, the additional waveform 406 is time-aligned with the filtered waveform 402 and the unfiltered waveform 404 on the display 400. However, the additional parameter waveform 406 may include a line that is distinguishable from the lines representing the filtered waveform 402 and the unfiltered waveform 404. For instance, the additional parameter waveform 406 may have a different emphasis than the filtered waveform 402 and the unfiltered waveform 404.

In various cases, a medical device may display filtered and unfiltered data simultaneously when one or more conditions have been satisfied. In some cases, the medical device displays unfiltered data when the condition(s) are unsatisfied and filtered data when the condition(s) are not satisfied. For instance, the medical device displays segments of unfiltered data and segments of unfiltered data, wherein the segments represent different time periods.

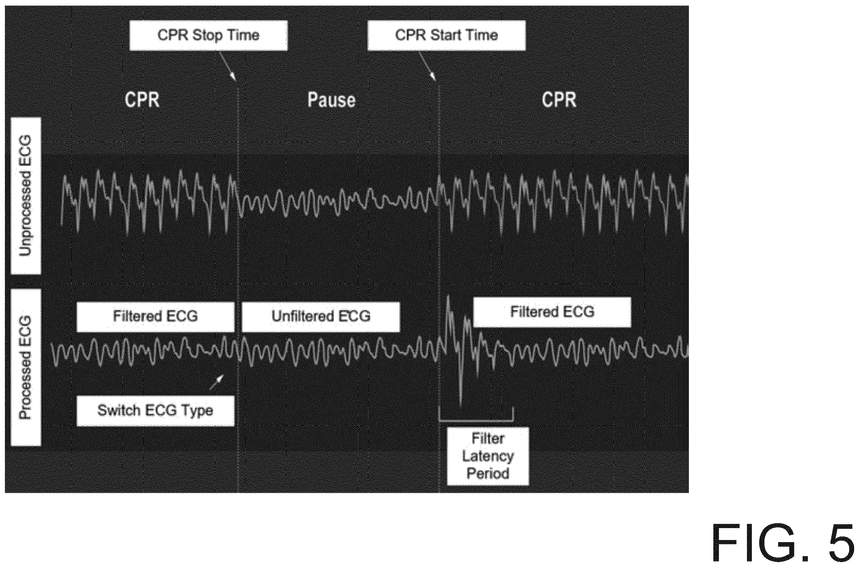

FIG. 5 illustrates an example of unprocessed ECG detected by a medical device and processed ECG displayed by the medical device. The unprocessed ECG is detected from a subject (e.g., a patient) receiving chest compressions during a first CPR period, is receiving no chest compressions during a pause period, and is receiving chest compressions during a second CPR period. The pause period occurs between the first CPR period and the second CPR period. The first CPR period ends at a CPR stop time. The second CPR period begins at a CPR start time. Thus, the unprocessed ECG includes a chest compression artifact during the first CPR period and a second CPR period.

The processed ECG includes three segments corresponding to the first CPR period, the pause period, and the second CPR period. In particular, a first segment of the processed ECG includes a first filtered ECG segment. The first filtered ECG segment is generated by applying a filter (e.g., a comb filter) to the segment of the unprocessed ECG detected during the first CPR period. A second segment of the processed ECG is identical to the segment of the unprocessed ECG occurring during the pause period. That is, the second segment of the processed ECG includes an unfiltered ECG segment. A third segment of the processed ECG includes a second filtered ECG segment. The second filtered ECG segment is generated by applying the filter to the segment of the unprocessed ECG detected during the second CPR period. However, an initial portion of the second filtered ECG segment includes an artifact caused by the latency period of the filter.

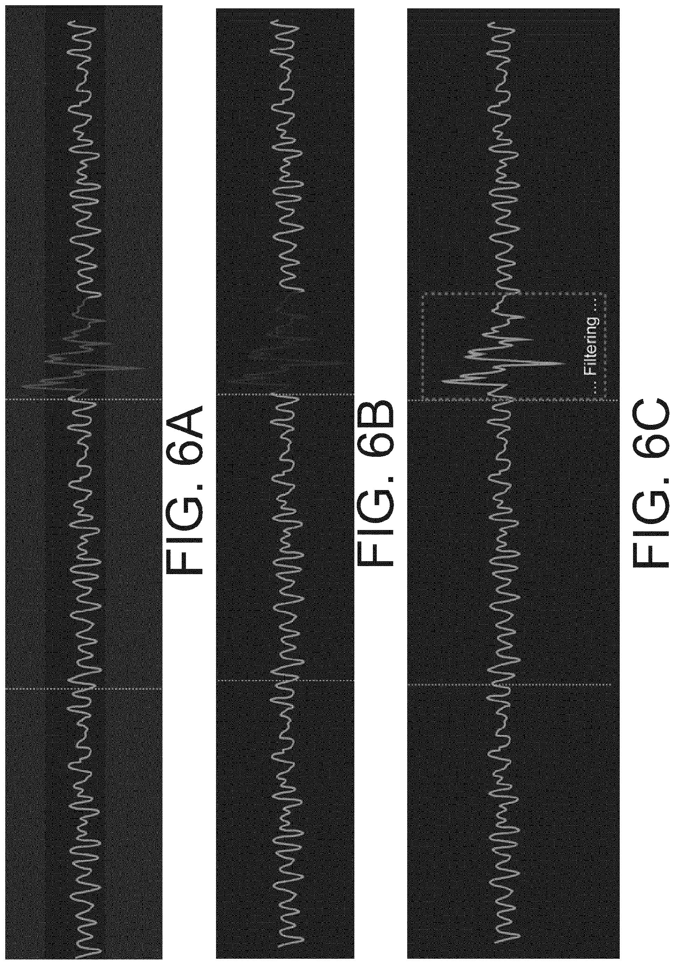

FIGS. 6A to 6C illustrate various examples of processed ECG displayed by a medical device. In various cases, FIGS. 6A to 6C provide alternative displays to the processed ECG illustrated above in FIG. 5. In particular cases, FIGS. 6A to 6C illustrate techniques for addressing the artifact caused by the latency period of the filtered in the second filtered ECG segment.

FIG. 6A illustrates an example of graying out the artifact caused by the latency period of the filter. By graying out the portion of the second filtered ECG segment that includes the latency artifact, a user may readily identify that the latency artifact is present in the portion.

FIG. 6B illustrates an example of reducing the contrast of the artifact caused by the latency period of the filter. By reducing the contrast of the portion of the second filtered ECG segment that includes the latency artifact, a user may readily identify that the latency artifact is present in the portion.

FIG. 6C illustrates an example of adding a user interface element that indicates the latency period of the filter. For example, a box can be displayed around the portion of the second filtered ECG segment that includes the latency artifact. In some cases, a label (e.g., "...filtering...") can be further displayed with the box that indicates the latency artifact. Accordingly, a user may readily identify that the latency artifact is present in the portion.

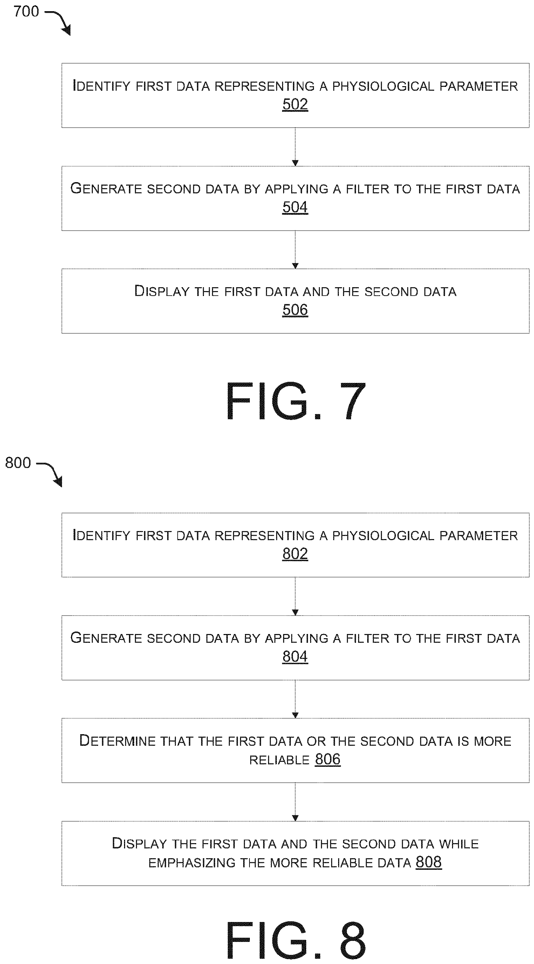

FIG. 7 illustrates an example process 700 for simultaneously displaying filtered and unfiltered data. The process 700 is performed by an entity, such as a medical device, the monitoring device 106, a processor executing instructions stored in memory, or any combination thereof.

At 702, the entity identifies first data representing a physiological parameter. For example, the entity includes a sensor that detects an electrocardiogram (ECG), a capnograph, a transthoracic impedance, a force administered to a user, a blood pressure, an airway parameter, a partial pressure of oxygen, an electroencephalogram (EEG), a temperature, a blood flow, or a pulse rate of a subject. In various implementations, the entity generates the first data by converting an analog signal detected by the sensor to digital data. In various cases, the first data includes an artifact. According to some examples, the artifact is generated by a treatment administered to the subject. For instance, the artifact is generated by chest compressions being administered to the subject. It is noted that even though the process 700 may be performed during treatment being administered to the subject, there is no functional link between the process 700 and any effects of a surgical or therapeutic nature that would occur during the administering of the treatment.

At 704, the entity generates second data by applying a filter to the first data. In various implementations, the filter is configured to at least partially remove the artifact. In some examples, the filter is a comb filter configured to reject a frequency of the artifact and one or more harmonics of the frequency. For instance, the device administers a treatment to the subject at the frequency. In some cases, the entity determines the frequency by analyzing the first data and/or receiving a report of the frequency from the device.

At 706, the entity displays the first data and the second data. In various implementations, the entity displays a first waveform representing the first data and a second waveform representing the second data. The first waveform is time-aligned with the second waveform on a display, for instance. In some cases, the first waveform and the second waveform are overlapping on the display. According to some implementations, the entity presents the waveforms at different emphases. For example, the waveforms have different colors, different contrasts, different line sizes, different transparencies, or different line styles. In some cases, the entity emphasizes the second waveform over the first waveform.

According to various implementations, the entity further detects one or more physiological events by analyzing the first data and/or the second data. The entity may visually present one or more symbols representing the physiological event(s) with the first data and the second data.

FIG. 8 illustrates an example process 800 for emphasizing one type of displayed data over another type of displayed data. The process 800 is performed by an entity, such as a medical device, the monitoring device 106, a processor executing instructions stored in memory, or any combination thereof.

At 802, the entity identifies first data representing a physiological parameter. For example, the entity includes a sensor that detects an electrocardiogram (ECG), a capnograph, a transthoracic impedance, a force administered to a user, a blood pressure, an airway parameter, a partial pressure of oxygen, an electroencephalogram (EEG), a temperature, a blood flow, or a pulse rate of a subject. In various implementations, the entity generates the first data by converting an analog signal detected by the sensor to digital data. In various cases, the first data includes an artifact. According to some examples, the artifact is generated by a treatment administered to the subject. For instance, the artifact is generated by chest compressions being administered to the subject.

At 804, the entity generates second data by applying a filter to the first data. In various implementations, the filter is configured to at least partially remove the artifact. The filter may be generated based on a treatment parameter. In some examples, the filter is a comb filter configured to reject a frequency of the artifact and one or more harmonics of the frequency. For instance, the device administers a treatment to the subject at the frequency. In some cases, the entity determines the frequency by analyzing the first data and/or receiving a report of the frequency from the device.

At 806, the entity determines that the first data or the second data is more reliable. For example, the entity may determine that the first data is more reliable than the second data if the treatment is not being administered to the subject, if the treatment has changed, if a latency period of the filter is occurring, or any combination thereof. In some cases, the entity determines that the second data is more reliable if the treatment is being administered to the subject, if the treatment has not changed within a threshold time period, if the latency period of the filter has expired, or any combination thereof. In some examples, the entity receives an input signal from a user indicating that the first data or the second data is more reliable.

At 808, the entity displays the first data and the second data while emphasizing the more reliable data. For example, the entity generates waveforms representing the first data and the second data. The waveform representing the more reliable data, for instance, has a bolder color, a larger contrast, a greater line size, a lower transparency, and/or a more solid line style than the waveform representing the less reliable data.

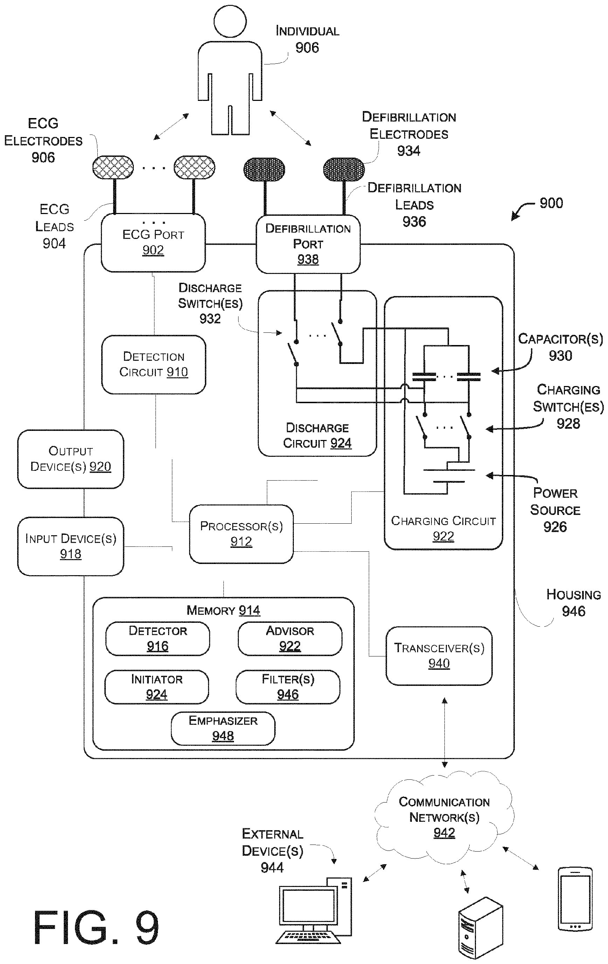

FIG. 9 illustrates an example of an external defibrillator 900 configured to perform various functions described herein. For example, the external defibrillator 900 is the monitoring device 106 or the treating device 110 described above with reference to FIG. 1.

The external defibrillator 900 includes an electrocardiogram (ECG) port 902 connected to multiple ECG leads 904. In some cases, the ECG leads 904 are removeable from the ECG port 902. For instance, the ECG leads 904 are plugged into the ECG port 902. The ECG leads 904 are connected to ECG electrodes 906, respectively. In various implementations, the ECG electrodes 906 are disposed on different locations on an individual 908. A detection circuit 910 is configured to detect relative voltages between the ECG electrodes 906. These voltages are indicative of the electrical activity of the heart of the individual 908.

In various implementations, the ECG electrodes 906 are in contact with the different locations on the skin of the individual 908. In some examples, a first one of the ECG electrodes 906 is placed on the skin between the heart and right arm of the individual 908, a second one of the ECG electrodes 906 is placed on the skin between the heart and left arm of the individual 908, and a third one of the ECG electrodes 906 is placed on the skin between the heart and a leg (either the left leg or the right leg) of the individual 908. In these examples, the detection circuit 908 is configured to measure the relative voltages between the first, second, and third ECG electrodes 906. Respective pairings of the ECG electrodes 906 are referred to as "leads," and the voltages between the pairs of ECG electrodes 906 are known as "lead voltages." In some examples, more than three ECG electrodes 906 are included, such that 5-lead or 12-lead ECG signals are detected by the detection circuit 910.

The detection circuit 910 includes at least one analog circuit, at least one digital circuit, or a combination thereof. The detection circuit 910 receives the analog electrical signals from the ECG electrodes 906, via the ECG port 902 and the ECG leads 904. In some cases, the detection circuit 910 includes one or more analog filters configured to filter noise and/or artifact from the electrical signals. The detection circuit 910 includes an analog-to-digital (ADC) in various examples. The detection circuit 910 generates a digital signal indicative of the analog electrical signals from the ECG electrodes 906. This digital signal can be referred to as an "ECG signal" or an "ECG."

In some cases, the detection circuit 910 further detects an electrical impedance between at least one pair of the ECG electrodes 906. For example, the detection circuit 910 includes, or otherwise controls, a power source that applies a known voltage (or current) across a pair of the ECG electrodes 906 and detects a resultant current (or voltage) between the pair of the ECG electrodes 906. The impedance is generated based on the applied signal (voltage or current) and the resultant signal (current or voltage). In various cases, the impedance corresponds to respiration of the individual 908, chest compressions performed on the individual 908, and other physiological states of the individual 908. In various examples, the detection circuit 910 includes one or more analog filters configured to filter noise and/or artifact from the resultant signal. The detection circuit 910 generates a digital signal indicative of the impedance using an ADC. This digital signal can be referred to as an "impedance signal" or an "impedance." In some cases, the detection circuit 910 includes additional components configured to detect electrical signals representing other types of physiological parameters described herein. One or more ADCs in the detection circuit 910 are further configured to convert the detected electrical signals into digital data.

The detection circuit 910 provides the ECG signal, the impedance signal, and/or signals representing other physiological parameters to one or more processors 912 in the external defibrillator 900. In some implementations, the processor(s) 912 includes a central processing unit (CPU), a graphics processing unit (GPU), both CPU and GPU, or other processing unit or component known in the art.

The processor(s) 912 is operably connected to memory 914. In various implementations, the memory 912 is volatile (such as random access memory (RAM)), non-volatile (such as read only memory (ROM), flash memory, etc.) or some combination of the two. The memory 914 stores instructions that, when executed by the processor(s) 912, causes the processor(s) 912 to perform various operations. In various examples, the memory 914 stores methods, threads, processes, applications, objects, modules, any other sort of executable instruction, or a combination thereof. In some cases, the memory 914 stores files, databases, or a combination thereof. In some examples, the memory 914 includes, but is not limited to, RAM, ROM, electrically erasable programmable read-only memory (EEPROM), flash memory, or any other memory technology. In some examples, the memory 914 includes one or more of CD-ROMs, digital versatile discs (DVDs), content-addressable memory (CAM), or other optical storage, magnetic cassettes, magnetic tape, magnetic disk storage or other magnetic storage devices, or any other medium which can be used to store the desired information and which can be accessed by the processor(s) 912 and/or the external defibrillator 900. In some cases, the memory 914 at least temporarily stores the ECG signal and/or the impedance signal.

In various examples, the memory 914 includes a detector 916, which causes the processor(s) 912 to determine, based on the ECG signal and/or the impedance signal, whether the individual 908 is exhibiting a particular heart rhythm. For instance, the processor(s) 912 determines whether the individual 908 is experiencing a shockable rhythm that is treatable by defibrillation. Examples of shockable rhythms include VF and pulseless VT. In some examples, the processor(s) 912 determines whether any of a variety of different rhythms (e.g., asystole, sinus rhythm, atrial fibrillation (AF), etc.) are present in the ECG signal. In various implementations, the detector 916, when executed by the processor(s) 912, further causes the processor(s) 912 to identify one or more physiological events in data representing various physiological parameters described herein.

The processor(s) 912 is operably connected to one or more input devices 918 and one or more output devices 920. Collectively, the input device(s) 918 and the output device(s) 920 function as an interface between a user and the defibrillator 900. The input device(s) 918 is configured to receive an input from a user and includes at least one of a keypad, a cursor control, a touch-sensitive display, a voice input device (e.g., a microphone), a haptic feedback device (e.g., a gyroscope), or any combination thereof. The output device(s) 920 includes at least one of a display, a speaker, a haptic output device, a printer, or any combination thereof. In various examples, the processor(s) 912 causes a display among the input device(s) 918 to visually output a waveform of the ECG signal and/or the impedance signal. In some implementations, the input device(s) 918 includes one or more touch sensors, the output device(s) 920 includes a display screen, and the touch sensor(s) are integrated with the display screen. Thus, in some cases, the external defibrillator 900 includes a touchscreen configured to receive user input signal(s) and visually output physiological parameters, such as the ECG signal and/or the impedance signal.

In some examples, the memory 914 includes an advisor 922, which, when executed by the processor(s) 912, causes the processor(s) 912 to generate advice and/or control the output device(s) 920 to output the advice to a user (e.g., a rescuer). In some examples, the processor(s) 912 provides, or causes the output device(s) 920 to provide, an instruction to perform CPR on the individual 908. In some cases, the processor(s) 912 evaluates, based on the ECG signal, the impedance signal, or other physiological parameters, CPR being performed on the individual 908 and causes the output device(s) 920 to provide feedback about the CPR in the instruction. According to some examples, the processor(s) 912, upon identifying that a shockable rhythm is present in the ECG signal, causes the output device(s) 920 to output an instruction and/or recommendation to administer a defibrillation shock to the individual 908.

The memory 914 also includes an initiator 924 which, when executed by the processor(s) 912, causes the processor(s) 912 to control other elements of the external defibrillator 900 in order to administer a defibrillation shock to the individual 908. In some examples, the processor(s) 912 executing the initiator 924 selectively controls the other elements of the external defibrillator 900 to cause the administration of the defibrillation shock based on determining that the individual 908 is exhibiting the shockable rhythm and/or based on an input from a user (received, e.g., by the input device(s) 918. In some cases, the processor(s) 912 controls the other elements of the external defibrillator 900 to cause the defibrillation shock to be output at a particular time, which is determined by the processor(s) 912 based on the ECG signal and/or the impedance signal.

In various implementations, the memory 914 further include one or more filters 946 which, when executed by the processor(s) 912, cause the processor(s) 912 to filter and/or remove artifact from one or more sets of data generated by the detection circuit 910. Further, the memory 914 stores an emphasizer 948 which, when executed by the processor(s) 912, cause the processor(s) 912 to cause a display in the output device(s) 920 to display one or more waveforms, symbols, and other visual signals described herein. The emphasizer 948 further causes the processor(s) 912 to determine whether one set of data is more reliable than another set of data, and causes the display to emphasize the more reliable set of data on the display.

The processor(s) 912 is operably connected to a charging circuit 922 and a discharge circuit 923. In various implementations, the charging circuit 922 includes a power source 926, one or more charging switches 928, and one or more capacitors 930. The power source 926 includes, for instance, a battery. The processor(s) 912 initiates a defibrillation shock by causing the power source 926 to charge at least one capacitor among the capacitor(s) 930. For example, the processor(s) 912 activates at least one of the charging switch(es) 928 in the charging circuit 922 to complete a first circuit connecting the power source 926 and the capacitor to be charged. Then, the processor(s) 912 causes the discharge circuit 923 to discharge energy stored in the charged capacitor across a pair of defibrillation electrodes 930, which are in contact with the individual 908. For example, the processor(s) 912 deactivates the charging switch(es) 928 completing the first circuit between the capacitor(s) 930 and the power source 926, and activates one or more discharge switches 932 completing a second circuit connecting the charged capacitor 930 and at least a portion of the individual 908 disposed between defibrillation electrodes 934.

The energy is discharged from the defibrillation electrodes 934 in the form of a defibrillation shock. For example, the defibrillation electrodes 934 are connected to the skin of the individual 908 and located at positions on different sides of the heart of the individual 908, such that the defibrillation shock is applied across the heart of the individual 908. The defibrillation shock, in various examples, depolarizes a significant number of heart cells in a short amount of time. The defibrillation shock, for example, interrupts the propagation of the shockable rhythm (e.g., VF or V-Tach) through the heart. In some examples, the defibrillation shock is 200 J or greater with a duration of about 0.015 seconds. In some cases, the defibrillation shock has a multiphasic (e.g., biphasic) waveform. The discharge switch(es) 932 are controlled by the processor(s) 912, for example. In various implementations, the defibrillation electrodes 934 are connected to defibrillation leads 936. The defibrillation leads 936 are connected to a defibrillation port 938, in implementations. According to various examples, the defibrillation leads 936 are removable from the defibrillation port 938. For example, the defibrillation leads 936 are plugged into the defibrillation port 938.

In various implementations, the processor(s) 912 is operably connected to one or more transceivers 940 that transmit and/or receive data over one or more communication networks 942. For example, the transceiver(s) 940 includes a network interface card (NIC), a network adapter, a local area network (LAN) adapter, or a physical, virtual, or logical address to connect to the various external devices and/or systems. In various examples, the transceiver(s) 940 includes any sort of wireless transceivers capable of engaging in wireless communication (e.g., radio frequency (RF) communication). For example, the communication network(s) 942 includes one or more wireless networks that include a 3^{rd} Generation Partnership Project (3GPP) network, such as a Long Term Evolution (LTE) radio access network (RAN) (e.g., over one or more LTE bands), a New Radio (NR) RAN (e.g., over one or more NR bands), or a combination thereof. In some cases, the transceiver(s) 940 includes other wireless modems, such as a modem for engaging in WI-FI^{®}, WIGIG^{®}, WIMAX^{®}, BLUETOOTH^{®}, or infrared communication over the communication network(s) 942.

The defibrillator 900 is configured to transmit and/or receive data (e.g., ECG data, impedance data, data indicative of one or more detected heart rhythms of the individual 908, data indicative of one or more defibrillation shocks administered to the individual 908, etc.) with one or more external devices 944 via the communication network(s) 942. The external devices 944 include, for instance, mobile devices (e.g., mobile phones, smart watches, etc.), Internet of Things (IoT) devices, medical devices, computers (e.g., laptop devices, servers, etc.), or any other type of computing device configured to communicate over the communication network(s) 942. In some examples, the external device(s) 944 is located remotely from the defibrillator 900, such as at a remote clinical environment (e.g., a hospital). According to various implementations, the processor(s) 912 causes the transceiver(s) 940 to transmit data to the external device(s) 944. In some cases, the transceiver(s) 940 receives data from the external device(s) 944 and the transceiver(s) 940 provide the received data to the processor(s) 912 for further analysis.

In various implementations, the external defibrillator 900 also includes a housing 946 that at least partially encloses other elements of the external defibrillator 900. For example, the housing 946 encloses the detection circuit 910, the processor(s) 912, the memory 914, the charging circuit 922, the transceiver(s) 940, or any combination thereof. In some cases, the input device(s) 918 and output device(s) 920 extend from an interior space at least partially surrounded by the housing 946 through a wall of the housing 946. In various examples, the housing 946 acts as a barrier to moisture, electrical interference, and/or dust, thereby protecting various components in the external defibrillator 900 from damage.

In some implementations, the external defibrillator 900 is an automated external defibrillator (AED) operated by an untrained user (e.g., a bystander, layperson, etc.) and can be operated in an automatic mode. In automatic mode, the processor(s) 912 automatically identifies a rhythm in the ECG signal, makes a decision whether to administer a defibrillation shock, charges the capacitor(s) 930, discharges the capacitor(s) 930, or any combination thereof. In some cases, the processor(s) 912 controls the output device(s) 920 to output (e.g., display) a simplified user interface to the untrained user. For example, the processor(s) 912 refrains from causing the output device(s) 920 to display a waveform of the ECG signal and/or the impedance signal to the untrained user, in order to simplify operation of the external defibrillator 900.

In some examples, the external defibrillator 900 is a monitor-defibrillator utilized by a trained user (e.g., a clinician, an emergency responder, etc.) and can be operated in a manual mode or the automatic mode. When the external defibrillator 900 operates in manual mode, the processor(s) 912 cause the output device(s) 920 to display a variety of information that may be relevant to the trained user, such as waveforms indicating the ECG data and/or impedance data, notifications about detected heart rhythms, and the like.

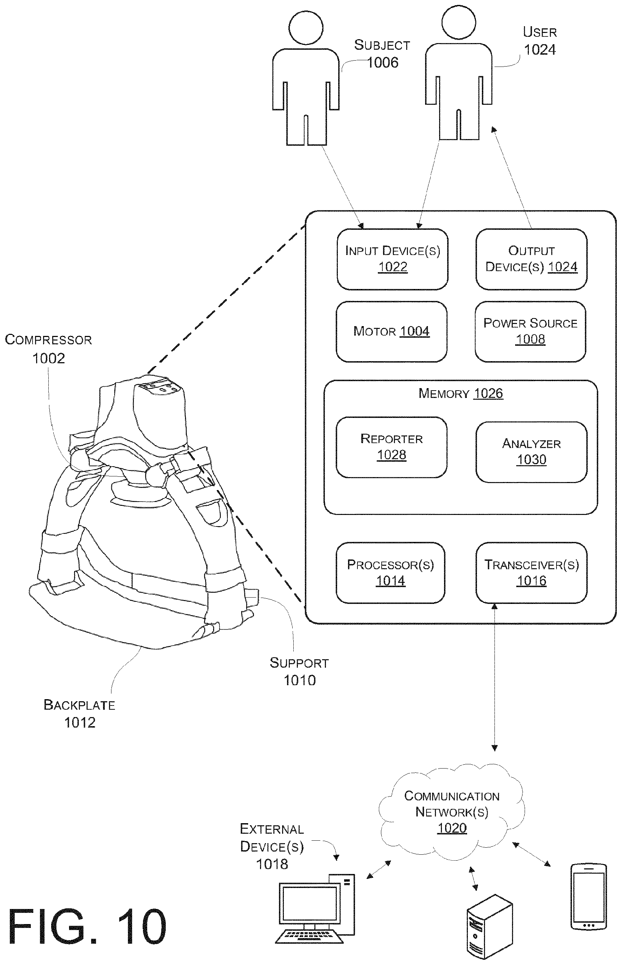

FIG. 10 illustrates a chest compression device 1000 configured to perform various functions described herein. For example, the chest compression device 1000 is the monitoring device 106 or the treating device 110 described in FIG. 1.

In various implementations, the chest compression device 1000 includes a compressor 1002 that is operatively coupled to a motor 1004. The compressor 1002 physically administers a force to the chest of a subject 1006 that compresses the chest of the subject 1006. In some examples, the compressor 1002 includes at least one piston that periodically moves between two positions (e.g., a compressed position and a release position) at a compression frequency. For example, when the piston is positioned on the chest of the subject 1006, the piston compresses the chest when the piston is moved into the compressed position. A suction cup may be positioned on a tip of the piston, such that the suction cup contacts the chest of the subject 1006 during operation. In various cases, the compressor 1002 includes a band that periodically tightens to a first tension and loosens to a second tension at a compression frequency. For instance, when the band is disposed around the chest of the subject 1006, the band compresses the chest when the band tightens.

The motor 1004 is configured to convert electrical energy stored in a power source 1008 into mechanical energy that moves and/or tightens the compressor 1002, thereby causing the compressor 1002 to administer the force to the chest of the subject 1006. In various implementations, the power source 1008 is portable. For instance, the power source 1008 includes at least one rechargeable (e.g., lithium-ion) battery. In some cases, the power source 1008 supplies electrical energy to one or more elements of the chest compression device 1000 described herein.

In various cases, the chest compression device 1000 includes a support 1010 that is physically coupled to the compressor 1002, such that the compressor 1002 maintains a position relative to the subject 1006 during operation. In some implementations, the support 1010 is physically coupled to a backplate 1012, cot, or other external structure with a fixed position relative to the subject 1006. According to some cases, the support 1010 is physically coupled to a portion of the subject 1006, such as wrists of the subject 1006.

The operation of the chest compression device 1000 may be controlled by at least one processor 1014. In various implementations, the motor 1006 is communicatively coupled to the processor(s) 1014. Specifically, the processor(s) 1014 is configured to output a control signal to the motor 1006 that causes the motor 1006 to actuate the compressor 1002. For instance, the motor 1006 causes the compressor 1002 to administer the compressions to the subject 1006 based on the control signal. In some cases, the control signal indicates one or more treatment parameters of the compressions. Examples of treatment parameters include a frequency, timing, depth, force, position, velocity, and acceleration of the compressor 1002 administering the compressions. According to various cases, the control signal causes the motor 1006 to cease compressions.

In various implementations, the chest compression device 1000 includes at least one transceiver 1016 configured to communicate with at least one external device 1018 over one or more communication networks 1020. Any communication network described herein can be included in the communication network(s) 1020 illustrated in FIG. 10. The external device(s) 1018, for example, includes at least one of a monitor-defibrillator, an AED, an ECMO device, a ventilation device, a patient monitor, a mobile phone, a server, or a computing device. In some implementations, the transceiver(s) 1016 is configured to communicate with the external device(s) 1018 by transmitting and/or receiving signals wirelessly. For example, the transceiver(s) 1016 includes a NIC, a network adapter, a LAN adapter, or a physical, virtual, or logical address to connect to the various external devices and/or systems. In various examples, the transceiver(s) 1016 includes any sort of wireless transceivers capable of engaging in wireless communication (e.g., RF communication). For example, the communication network(s) 1020 includes one or more wireless networks that include a 3GPP network, such as an LTE RAN (e.g., over one or more LTE bands), an NR RAN (e.g., over one or more NR bands), or a combination thereof. In some cases, the transceiver(s) 1016 includes other wireless modems, such as a modem for engaging in WI-FI^{®}, WIGIG^{®}, WIMAX^{®}, BLUETOOTH^{®}, or infrared communication over the communication network(s) 1020. The signals, in various cases, encode data in the form of data packets, datagrams, or the like. In some cases, the signals are transmitted as compressions are being administered by the chest compression device 1000 (e.g., for real-time feedback by the external device(s) 1018), after compressions are administered by the chest compression device 1000 (e.g., for post-event review at the external device 1018), or a combination thereof.

In various cases, the processor(s) 1014 generates the control signal based on data encoded in the signals received from the external device(s) 1018. For instance, the signals include an instruction to initiate the compressions, and the processor(s) 1014 instructs the motor 1006 to begin actuating the compressor 1002 in accordance with the signals.

In some cases, the chest compression device 1000 includes at least one input device 1022. In various examples, the input device(s) 1022 is configured to receive an input signal from a user 1024, who may be a rescuer treating the subject 1006. Examples of the input device(s) 1022 include, for instance, at a keypad, a cursor control, a touch-sensitive display, a voice input device (e.g., a microphone), a haptic feedback device (e.g., a gyroscope), or any combination thereof. In various implementations, the processor(s) 1014 generate the control signal based on the input signal. For instance, the processor(s) 1014 generate the control signal to adjust a frequency of the compressions based on the chest compression device 1000 detecting a selection by the user 1024 of a user interface element displayed on a touchscreen or detecting the user 1024 pressing a button integrated with an external housing of the chest compression device 1000.

According to some examples, the input device(s) 1022 include one or more sensors. The sensor(s), for example, is configured to detect a physiological parameter of the subject 1006. In some implementations, the sensor(s) is configured to detect a state parameter of the chest compression device 1000, such as a position of the compressor 1002 with respect to the subject 1006 or the backplate 1012, a force administered by the compressor 1002 on the subject 1006, a force administered onto the backplate 1022 by the body of the subject 1006 during a compression, or the like. According to some implementations, the signals transmitted by the transceiver(s) 1016 indicate the physiological parameter(s) and/or the state parameter(s).

The chest compression device 1000 further includes at least one output device 1024, in various implementations. Examples of the output device(s) 1024 include, for instance, least one of a display (e.g., a projector, an LED screen, etc.), a speaker, a haptic output device, a printer, or any combination thereof. In some implementations, the output device(s) 1024 include a screen configured to display various parameters detected by and/or reported to the chest compression device 1000, a charge level of the power source 1008, a timer indicating a time since compressions were initiated or paused, and other relevant information.

The chest compression device 1000 further includes memory 1026. In various implementations, the memory 1026 is volatile (such as random access memory (RAM)), non-volatile (such as read only memory (ROM), flash memory, etc.) or some combination of the two. The memory 1026 stores instructions that, when executed by the processor(s) 1014, causes the processor(s) 1014 to perform various operations. In various examples, the memory 1026 stores methods, threads, processes, applications, objects, modules, any other sort of executable instruction, or a combination thereof. In some cases, the memory 1026 stores files, databases, or a combination thereof. In some examples, the memory 1026 includes, but is not limited to, RAM, ROM, EEPROM, flash memory, or any other memory technology. In some examples, the memory 1026 includes one or more of CD-ROMs, DVDs, CAM, or other optical storage, magnetic cassettes, magnetic tape, magnetic disk storage or other magnetic storage devices, or any other medium which can be used to store the desired information. In various cases, the memory 1026 stores instructions, programs, threads, objects, data, or any combination thereof, that cause the processor(s) 1014 to perform various functions. In various cases, the memory 1026 stores one or more parameters that are detected by the chest compression device 1000 and/or reported to the chest compression device 1000.

In implementations of the present disclosure, the memory 1026 also stores instructions for executing a reporter 1028 and analyzer 1030. The processor(s) 1014, when executing the reporter 1028, may be configured to generate communication signals to communicate treatment parameters to an external device, wherein the communication signals are output by the transceiver(s) 1016. In various cases, the analyzer 1030 causes the processor(s) 1014 to analyze data generated by the input device(s) 1022.

### EXAMPLE CLAUSES

1. A monitor-defibrillator, including: a detection circuit configured to detect an electrocardiogram (ECG) signal of a subject; an analog-to-digital converter (ADC) configured to convert the ECG signal into unfiltered ECG data; a transceiver configured to receive a signal from a mechanical chest compression device, the signal indicating a frequency at which the mechanical chest compression device is administering chest compressions to the subject; a display; and a processor configured to: generate a comb filter that rejects the frequency and harmonics of the frequency; generate filtered ECG data by applying the comb filter to the unfiltered ECG data; cause the display to visually present the unfiltered ECG data at a first emphasis; and cause the display to visually present the filtered ECG data at a second emphasis, the second emphasis being greater than the first emphasis.
2. The monitor-defibrillator of clause 1, the filtered ECG data being first filtered ECG data, the processor being further configured to: determine, by analyzing the unfiltered ECG data or the filtered ECG data, a transition between chest compressions administered by the mechanical chest compression device to chest compressions administered by a rescuer; generate second filtered ECG data by applying an alternate filter to the unfiltered ECG data detected during the chest compressions administered by the rescuer; and cause the display to visually present the second filtered ECG data at a third emphasis, the third emphasis being different than the second emphasis.
3. The monitor-defibrillator of clause 1 or 2, the processor being further configured to: detect, by analyzing the unfiltered ECG data or the filtered ECG data, a chest compression artifact in the unfiltered ECG data; and cause the display to visually present a symbol indicating a time at which the chest compression artifact is in the unfiltered ECG data.
4. A method performed by a first medical device, the method including: detecting an analog signal indicating a physiological parameter of a subject; converting the analog signal to first data; determining a treatment parameter characterizing a treatment administered to the subject by a second medical device; generating a filter characterized by the treatment parameter; generating second data by applying the filter to the first data; and displaying, on a user interface, the first data and the second data, wherein the second data is emphasized on the user interface.
5. The method of clause 4, wherein the physiological parameter includes an electrocardiogram (ECG), a capnograph, a transthoracic impedance, a force administered to a user, a blood pressure, an airway parameter, a partial pressure of oxygen, an electroencephalogram (EEG), a temperature, a blood flow, or a pulse rate.
6. The method of clause 4 or 5, wherein determining the treatment parameter includes: determining, by analyzing the first data, a frequency of the treatment.
7. The method of one of clauses 4 to 6, wherein determining the treatment parameter includes: receiving, from the second medical device, a signal indicating the treatment parameter.
8. The method of one of clauses 4 to 7, further including: determining that the second data is more reliable than the first data.
9. The method of one of clauses 4 to 8, wherein displaying the first data and the second data includes overlaying and time-aligning the first data and the second data on the user interface.
10. The method of one of clauses 4 to 9, wherein displaying the first data and the second data includes: displaying a first waveform representing the first data; and displaying a second waveform representing the second data.
11. The method of clause 10, wherein the first waveform includes a different color than the second waveform, the first waveform includes a different contrast than the second waveform, the first waveform includes a different line size than the second waveform, the first waveform includes a different transparency than the second waveform, and/or the first waveform includes a different line style than the second waveform.
12. The method of one of clauses 4 to 11, the physiological parameter including a first physiological parameter, the method further including: detecting a second physiological parameter of the subject; generating third data indicative of the second physiological parameter; and displaying, on the user interface, a waveform representing the third data.
13. The method of one of clauses 4 to 12, further including: detecting, by analyzing the first data or the second data, an event indicative of the treatment; and displaying, on the user interface, a symbol indicating the event.
14. An external defibrillator including: a discharge circuit configured to administer a first treatment to a subject; a sensor configured to detect a physiological parameter of the subject; a display; and a processor configured to: generate first data indicating the physiological parameter of the subject; determine a treatment parameter characterizing a second treatment administered to the subject; generate a filter characterized by the treatment parameter; generate second data by applying the filter to the first data; and cause the display to visually present the first data at a first emphasis and the second data at a second emphasis.
15. The external defibrillator of clause 14, wherein causing the display to visually present the first data and the second data includes: causing the display to visually present a first waveform representing the first data; and causing the display to visually present a second waveform representing the second data.
16. The external defibrillator of clause 15, wherein the second emphasis is greater than the first emphasis, and wherein the first waveform includes a different color than the second waveform, the first waveform includes a smaller contrast than the second waveform, the first waveform includes a smaller line size than the second waveform, the first waveform includes a greater transparency than the second waveform, and/or the first waveform includes a different line style than the second waveform.
17. The external defibrillator of one of clauses 14 to 16, wherein the treatment parameter includes a frequency of the treatment, a time at which the treatment begins being administered to the subject, or a time at which the treatment stops being administered to the subject.
18. The external defibrillator of one of clauses 14 to 17, the processor being further configured to: detect, by analyzing the first data or the second data, an event including a chest compression, a ventilation, or a QRS sense event; and display, on the user interface, a symbol indicating the event.
19. The external defibrillator of one of clauses 14 to 18, further including: a transceiver configured to receive, from a medical device administering the second treatment, a signal indicating the treatment parameter.
20. The external defibrillator of one of clauses 14 to 19, wherein the processor is configured to determine the treatment parameter by analyzing the first data or the second data.
21. A monitor-defibrillator, including: a detection circuit configured to detect an electrocardiogram (ECG) signal of a subject; an analog-to-digital converter (ADC) configured to convert the ECG signal into unfiltered ECG data; a transceiver configured to receive a signal from a mechanical chest compression device, the signal indicating a frequency at which the mechanical chest compression device is administering chest compressions to the subject; a display configured to visually present a user interface element corresponding to an ECG lead; an input device configured to receive an input signal from a user; and a processor configured to: generate a comb filter that rejects the frequency and harmonics of the frequency; generate filtered ECG data by applying the comb filter to the unfiltered ECG data; cause the display to visually present the unfiltered ECG data in the user interface element at a first emphasis; cause the display to visually present the filtered ECG data in the user interface element at a second emphasis, the second emphasis being greater than the first emphasis; and in response to the input device receiving the input signal from the user: cause the display to visually present the unfiltered ECG data in the user interface element at a third emphasis; and cause the display to visually present the filtered ECG data in the user interface element at a fourth emphasis, the third emphasis being greater than the fourth emphasis.
22. The monitor-defibrillator of clause 21, wherein the display is configured to overlay and time-align the filtered ECG data and the unfiltered ECG data in the user interface element.
23. The monitor-defibrillator of clause 21 or 22, wherein the processor is further configured to: detect, based on the unfiltered ECG data or the filtered ECG data, an event including a chest compression, a ventilation, or a QRS sense event; and cause the display to visually present a symbol indicating the event.
24. The monitor-defibrillator of one of clauses 21 to 23, wherein the processor is further configured to: cause the display to visually present a selectable element; and in response to the input device receiving a second input signal associated with the selectable element, cause the display to: visually present the filtered ECG data without the unfiltered ECG data; or visually present the unfiltered ECG data without the filtered ECG data.
25. The monitor-defibrillator of one of clauses 21 to 24, wherein the processor is further configured to: detect a filter artifact in the filtered ECG data; and cause the display to visually present: the filter artifact at a fifth emphasis; or an indication of the filter artifact.
26. The monitor-defibrillator of one of clauses 21 to 25, wherein causing the display to visually present the unfiltered ECG data at the third emphasis includes causing the display to visually present a first waveform representing the unfiltered ECG, and wherein causing the display to visually present the filtered ECG data at the fourth emphasis includes causing the display to visually present a second waveform representing the filtered ECG, the second waveform having a smaller line size than the first waveform.
27. A method including: detecting an analog signal indicating a physiological parameter of a subject; converting the analog signal to first data; determining a treatment parameter characterizing a treatment administered to the subject; generating a filter characterized by the treatment parameter; generating second data by applying the filter to the first data; displaying the first data at a first emphasis; displaying the second data at a second emphasis, the second emphasis being greater than the first emphasis; determining that the first data is more reliable than the second data; and in response determining that the first data is more reliable than the second data: displaying the first data at the second emphasis; and displaying the second data at the first emphasis.
28. The method of clause 27, wherein the physiological parameter includes an electrocardiogram (ECG) of the subject, and wherein the treatment includes chest compressions administered to the subject.
29. The method of clause 27 or 28, wherein determining that the first data is more reliable than the second data includes determining that an artifact associated with the treatment is absent from the first data.
30. The method of one of clauses 27 to 28, wherein displaying the first data at the first emphasis includes displaying a first waveform representing the first data; and wherein displaying the second data at the second emphasis includes displaying a second waveform representing the second data.
31. The method of clause 30, wherein the first waveform includes a different color than the second waveform, the first waveform includes a different contrast than the second waveform, the first waveform includes a different line size than the second waveform, the first waveform includes a different transparency than the second waveform, and/or the first waveform includes a different line style than the second waveform.
32. The method of one of clauses 27 to 31, wherein the treatment parameter includes a frequency of the treatment, a time at which the treatment began being administered to the subject, or a time at which the treatment stops being administered to the subject.
33. The method of one of clauses 27 to 32, further including: detecting, by analyzing the first data or the second data, an event including a chest compression, a ventilation, or a QRS sense event; and displaying, on the user interface, a symbol or a letter indicating the event.
34. A medical device, including: a sensor configured to detect an analog signal indicating a physiological parameter of a subject; an analog-to-digital converter (ADC) configured to convert the analog signal to first data; and a processor configured to: generate a filter configured to remove an artifact associated with a treatment administered to the subject; generate second data by applying the filter to the first data; determine that the first data is more reliable than the second data; and in response to determining that the first data is more reliable than the second data: cause a user interface to visually present a first waveform representing the first data at a first emphasis; and cause the user interface to visually present a second waveform representing the second data at a second emphasis, the first emphasis being greater than the second emphasis.
35. The medical device of clause 34, wherein the physiological parameter includes an electrocardiogram (ECG), a capnograph, a force administered to the subject, a blood pressure, an airway parameter, a partial pressure of oxygen, an electroencephalogram (EEG), a temperature, a blood flow, a pulse rate, or a transthoracic impedance.
36. The medical device of clause 34 or 35, wherein the processor is further configured for: detecting, by analyzing the first data or the second data, an event including a chest compression, a ventilation, or a QRS sense event; and causing the user interface to display a symbol indicating the event.
37. The medical device of one of clauses 34 to 36, wherein the treatment administered to the subject is administered by another medical device.
38. The medical device of one of clauses 34 to 37, the sensor being a first sensor, the physiological parameter being a first physiological parameter, the medical device further including: a second sensor configured to detect a second physiological parameter, wherein the processor is further configured to cause the user interface to visually present third data representing the second physiological parameter.
39. The medical device of clause 38, wherein the processor is configured to cause the user interface to visually present the third data by visually presenting a third waveform at a third emphasis.
40. The method of clause 39, wherein the first waveform includes a different color than the second waveform and the third waveform, the first waveform includes a different contrast than the second waveform and the third waveform, the first waveform includes a different line size than the second waveform and the third waveform, the first waveform includes a different transparency than the second waveform and the third waveform, and/or the first waveform includes a different line style than the second waveform and the third waveform.
41. A method, including: generating filtered data by removing a treatment artifact from unfiltered data, the unfiltered data representing a physiological parameter of a subject; displaying a segment of the filtered data corresponding to a treatment time interval, a treatment being administered to the subject during the treatment time interval; and displaying a segment of the unfiltered data corresponding to a non-treatment time interval, the treatment being paused during the non-treatment time interval.
42. The method of clause 41, further including: identifying a filter artifact in the segment of the filtered data corresponding to the treatment time interval, wherein displaying the segment of the filtered data corresponding to the treatment time interval includes indicating the filter artifact.

The features disclosed in the foregoing description, or the following claims, or the accompanying drawings, expressed in their specific forms or in terms of a means for performing the disclosed function, or a method or process for attaining the disclosed result, as appropriate, may, separately, or in any combination of such features, be used for realizing implementations of the disclosure in diverse forms thereof.

As will be understood by one of ordinary skill in the art, each implementation disclosed herein can comprise, consist essentially of or consist of its particular stated element, step, or component. Thus, the terms "include" or "including" should be interpreted to recite: "comprise, consist of, or consist essentially of." The transition term "comprise" or "comprises" means has, but is not limited to, and allows for the inclusion of unspecified elements, steps, ingredients, or components, even in major amounts. The transitional phrase "consisting of" excludes any element, step, ingredient or component not specified. The transition phrase "consisting essentially of" limits the scope of the implementation to the specified elements, steps, ingredients or components and to those that do not materially affect the implementation. As used herein, the term "based on" is equivalent to "based at least partly on," unless otherwise specified.

Unless otherwise indicated, all numbers expressing quantities, properties, conditions, and so forth used in the specification and claims are to be understood as being modified in all instances by the term "about." Accordingly, unless indicated to the contrary, the numerical parameters set forth in the specification and attached claims are approximations that may vary depending upon the desired properties sought to be obtained by the present disclosure. At the very least, and not as an attempt to limit the application of the doctrine of equivalents to the scope of the claims, each numerical parameter should at least be construed in light of the number of reported significant digits and by applying ordinary rounding techniques. When further clarity is required, the term "about" has the meaning reasonably ascribed to it by a person skilled in the art when used in conjunction with a stated numerical value or range, i.e. denoting somewhat more or somewhat less than the stated value or range, to within a range of ±20% of the stated value; ±19% of the stated value; ±18% of the stated value; ±17% of the stated value; ±16% of the stated value; ±15% of the stated value; ±14% of the stated value; ±13% of the stated value; ±12% of the stated value; ±11% of the stated value; ±10% of the stated value; ±9% of the stated value; ±8% of the stated value; ±7% of the stated value; ±6% of the stated value; ±5% of the stated value; t4% of the stated value; t3% of the stated value; ±2% of the stated value; or ±1% of the stated value.

Notwithstanding that the numerical ranges and parameters setting forth the broad scope of the disclosure are approximations, the numerical values set forth in the specific examples are reported as precisely as possible. Any numerical value, however, inherently contains certain errors necessarily resulting from the standard deviation found in their respective testing measurements.

The terms "a," "an," "the" and similar referents used in the context of describing implementations (especially in the context of the following claims) are to be construed to cover both the singular and the plural, unless otherwise indicated herein or clearly contradicted by context. Recitation of ranges of values herein is merely intended to serve as a shorthand method of referring individually to each separate value falling within the range. Unless otherwise indicated herein, each individual value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein is intended merely to better illuminate implementations of the disclosure and does not pose a limitation on the scope of the disclosure. No language in the specification should be construed as indicating any non-claimed element essential to the practice of implementations of the disclosure.

Groupings of alternative elements or implementations disclosed herein are not to be construed as limitations. Each group member may be referred to and claimed individually or in any combination with other members of the group or other elements found herein. It is anticipated that one or more members of a group may be included in, or deleted from, a group for reasons of convenience and/or patentability. When any such inclusion or deletion occurs, the specification is deemed to contain the group as modified thus fulfilling the written description of all Markush groups used in the appended claims.

Certain implementations are described herein, including the best mode known to the inventors for carrying out implementations of the disclosure. Of course, variations on these described implementations will become apparent to those of ordinary skill in the art upon reading the foregoing description. The inventors expect skilled artisans to employ such variations as appropriate, and the inventors intend for implementations to be practiced otherwise than specifically described herein. Accordingly, the scope of this disclosure includes all modifications and equivalents of the subject matter recited in the claims appended hereto as permitted by applicable law. Moreover, any combination of the above-described elements in all possible variations thereof is encompassed by implementations of the disclosure unless otherwise indicated herein or otherwise clearly contradicted by context.

## Claims

1. A method performed by a first medical device, the method comprising:
detecting an analog signal indicating a physiological parameter of a subject;
converting the analog signal to first data;
determining a treatment parameter characterizing a treatment administered to the subject by a second medical device;
generating a filter **characterized by** the treatment parameter;
generating second data by applying the filter to the first data; and
displaying, on a user interface, the first data and the second data, wherein the second data is emphasized on the user interface.

2. The method of claim 1, wherein the physiological parameter comprises an electrocardiogram (ECG), a capnograph, a transthoracic impedance, a force administered to a user, a blood pressure, an airway parameter, a partial pressure of oxygen, an electroencephalogram (EEG), a temperature, a blood flow, or a pulse rate.

3. The method of claim 1 or 2, wherein determining the treatment parameter comprises:
determining, by analyzing the first data, a frequency of the treatment; and/or
receiving, from the second medical device, a signal indicating the treatment parameter.

4. The method of any of claims 1-3, further comprising:
determining that the second data is more reliable than the first data.

5. The method of any of claims 1-4, wherein displaying the first data and the second data comprises overlaying and time-aligning the first data and the second data on the user interface.

6. The method of any of claims 1-5, wherein displaying the first data and the second data comprises:
displaying a first waveform representing the first data; and
displaying a second waveform representing the second data;
wherein optionally the first waveform comprises a different color than the second waveform, the first waveform comprises a different contrast than the second waveform, the first waveform comprises a different line size than the second waveform, the first waveform comprises a different transparency than the second waveform, and/or the first waveform comprises a different line style than the second waveform.

7. The method of any of claims 1-6, the physiological parameter comprising a first physiological parameter, the method further comprising:
detecting a second physiological parameter of the subject;
generating third data indicative of the second physiological parameter; and
displaying, on the user interface, a waveform representing the third data.

8. The method of any of claims 1-7, further comprising:
detecting, by analyzing the first data or the second data, an event indicative of the treatment; and
displaying, on the user interface, a symbol indicating the event.

9. The method of any of claims 1-8, wherein the first medical device is an external defibrillator.

10. An external defibrillator comprising:
a discharge circuit configured to administer a first treatment to a subject;
a sensor configured to detect a physiological parameter of the subject;
a display; and
a processor configured to:
generate first data indicating the physiological parameter of the subject;
determine a treatment parameter characterizing a second treatment administered to the subject;
generate a filter **characterized by** the treatment parameter;
generate second data by applying the filter to the first data; and
cause the display to visually present the first data at a first emphasis and the second data at a second emphasis.

11. The external defibrillator of claim 10, wherein the processor is configured to cause the display to visually present the first data and the second data by:
causing the display to visually present a first waveform representing the first data; and
causing the display to visually present a second waveform representing the second data; wherein optionally the second emphasis is greater than the first emphasis, and
wherein further optionally the first waveform comprises a different color than the second waveform, the first waveform comprises a smaller contrast than the second waveform, the first waveform comprises a smaller line size than the second waveform, the first waveform comprises a greater transparency than the second waveform, and/or the first waveform comprises a different line style than the second waveform.

12. The external defibrillator of claim 10 or 11, wherein the treatment parameter comprises a frequency of the treatment, a time at which the treatment begins being administered to the subject, or a time at which the treatment stops being administered to the subject.

13. The external defibrillator of any of claims 10-12, the processor being further configured to:
detect, by analyzing the first data or the second data, an event comprising a chest compression, a ventilation, or a QRS sense event; and
display, on the user interface, a symbol indicating the event.

14. The external defibrillator of any of claims 10-13, further comprising:
a transceiver configured to receive, from a medical device administering the second treatment, a signal indicating the treatment parameter.

15. The external defibrillator of any of claims 10-14, wherein the processor is configured to determine the treatment parameter by analyzing the first data or the second data.
